# FASCICULE DE BREVET EUROPEEN

(11) **EP 3 983 451 B1**
(45) Date de publication et mention de la délivrance du brevet: **02.08.2023**
(21) Numéro de dépôt: 20733231.3
(22) Date de dépôt: 10.06.2020
(51) Int. Cl.: C08C 19/22, C07D 233/61, C08F 236/10

(54) **POLYMÈRE GREFFÉ PORTANT DES GROUPES PENDANTS FONCTIONNELS IMIDAZOLE**
GEPFROPFTES POLYMER MIT ANHÄNGENDEN IMIDAZOLFUNKTIONSGRUPPEN
GRAFTED POLYMER CARRYING PENDANT IMIDAZOLE FUNCTIONAL GROUPS

(30) Priorité: 11.06.2019 FR 1906182
(43) Date de publication de la demande: 20.04.2022
(73) Titulaire: COMPAGNIE GENERALE DES ETABLISSEMENTS MICHELIN, 63000 Clermont-Ferrand (FR)
(72) Inventeur: JEAN-BAPTISTE DIT DOMINIQUE, François, 63040 CLERMONT-FERRAND Cedex 9 (FR)
(74) Mandataire: Bocchi, Brigitte
(86) Numéro de dépôt international: PCT/EP2020/066121
(87) Numéro de publication internationale: WO 2020/249631

(56) Documents cités:
- WO-A1-2015/059269
- WO-A1-2015/059271

## Description

### I-DOMAINE DE L'INVENTION

La présente invention concerne de nouveaux polymères fonctionnalisés par greffage d'un composé 1,3-dipolaire comprenant un cycle hétéroaromatique et une fonction imidazole. L'invention concerne également un procédé d'obtention de tels polymères.

### II-ARRIERE PLAN TECHNOLOGIQUE

La modification de structure d'un polymère, telle que sa fonctionnalisation par greffage, est particulièrement recherchée lorsque l'on souhaite mettre en présence un polymère et une charge dans une composition. Cette modification peut permettre d'améliorer, par exemple, la dispersion de la charge dans une matrice polymérique aboutissant ainsi à un matériau davantage homogène. *In fine,* les propriétés de la composition sont améliorées.

Dans le cas de certaines charges, comme les charges renforçantes telles que le noir de carbone ou la silice, une meilleure dispersion de cette charge va généralement se traduire par une baisse d'hystérèse de la composition. Une telle propriété est recherchée, notamment dans les compositions de caoutchouc destinées par exemple à des applications pour pneumatique. En effet, la baisse d'hystérèse d'une composition de caoutchouc est favorable à la diminution de la résistance au roulement d'un pneumatique et donc à une baisse de la consommation du carburant d'un véhicule roulant avec de tels pneumatiques.

Or, il est connu qu'une baisse d'hystérèse s'accompagne souvent d'une baisse de la rigidité à cuit de la composition de caoutchouc, ce qui peut rendre, dans certains cas, la composition inadaptée à l'usage que l'on veut en faire.

Il est donc souhaitable de disposer de polymères favorisant la dispersion de la charge renforçante sans provoquer une baisse excessive de la rigidité à cuit d'une composition de caoutchouc.

On connaît du document WO2015059269A1 des élastomères greffés par un composé de formule Q-A-B dans lequel le groupement Q comprend un dipôle contenant au moins un atome d'azote, A est un groupe divalent qui peut être aromatique ou non et B une fonction imidazole. Lorsque cet élastomère greffé est mélangé à des charges renforçantes dans une composition de caoutchouc, le compromis rigidité à cuit et hystérèse de cette composition de caoutchouc est amélioré par rapport à des compositions de caoutchouc ne comprenant pas d'élastomère greffé. Ces élastomères greffés sont donc particulièrement intéressants.

Le Demandeur a poursuivi ses recherches et a cherché à améliorer l'obtention de polymères greffés par un composé comprenant un dipôle contenant au moins un atome d'azote et ayant une fonction imidazole.

Après de nombreux essais, le Demandeur a découvert qu'il peut obtenir des polymères greffés portant des groupes pendants fonctionnels imidazole avec un rendement de greffage amélioré par rapport aux composés de l'art antérieur et plus rapidement en utilisant une famille de composés 1,3-dipolaire particuliers.

### III-RESUME DE L'INVENTION

L'invention a donc pour objet un polymère modifié obtenu par greffage d'au moins un composé de formule (I) sur au moins une insaturation de la chaîne d'un polymère initial 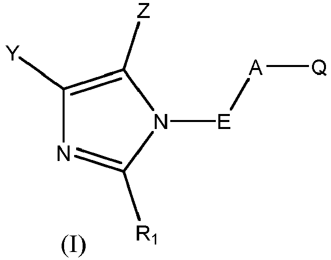 dans laquelle :
- Q représente un dipôle comprenant au moins un atome d'azote ;
- A représente un cycle hétéroaromatique divalent éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ; et
- Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent.

Avantageusement, le composé de formule (I) permet d'obtenir des polymères modifiés par greffage et portant des groupes pendants fonctionnels imidazole plus rapidement et avec un meilleur rendement que les composés de l'art antérieur.

Dans la présente, sauf indication expresse différente, tous les pourcentages (%) indiqués sont des pourcentages (%) en masse.

D'autre part, tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de a jusqu'à b (c'est-à-dire incluant les bornes strictes a et b). Dans la présente, lorsqu'on désigne un intervalle de valeurs par l'expression « de a à b », on désigne également et préférentiellement l'intervalle représenté par l'expression « entre a et b ».

Par « hétéroatome », sauf mention contraire, on entend un atome choisi dans le groupe constitué par un atome de soufre, un atome d'oxygène et un atome de d'azote.

Les composés comprenant du carbone mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont concernés notamment les polymères, les plastifiants, les charges, etc.

L'invention ainsi que ses avantages seront aisément compris à la lumière de la description et des exemples de réalisations qui suivent.

### IV-DESCRIPTION DETAILLEE DE L'INVENTION :

Comme expliqué précédemment, l'invention a donc pour objet un polymère modifié obtenu par greffage d'au moins un composé de formule (I) sur au moins une insaturation de la chaîne d'un polymère initial 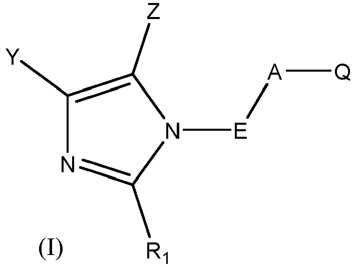 dans laquelle :
- Q représente un dipôle comprenant au moins un atome d'azote ;
- A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ; et
- Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent.

Par « polymère modifié obtenu par greffage » ou « polymère modifié par greffage », on entend un polymère comportant des fonctions, notamment des fonctions imidazoles, qui ont été introduites dans la chaîne du polymère. En pratique, le polymère modifié est obtenu par réaction de greffage d'un composé portant des fonctions imidazoles et portant une fonction apte à former une liaison covalente avec une insaturation de la chaîne du polymère, cette fonction étant un dipôle comprenant au moins un atome d'azote, de préférence une nitrone. Lors de la réaction de greffage, le dipôle comprenant au moins un atome d'azote du composé à greffer forme des liaisons covalentes avec une insaturation de la chaîne du polymère.

De manière connue, un polymère comprend généralement au moins une chaîne polymère principale. Cette chaîne polymère peut être qualifié de principale à partir du moment où toutes les autres chaînes du polymère sont considérées comme des chaînes pendantes comme le mentionne le document « Glossary of basic terms in polymer science » (IUPAC recommendations 1996), PAC, 1996, 68, 2287, p2294.

Par « insaturation » on entend une liaison covalente multiple entre deux atomes de carbone ; cette liaison covalente multiple pouvant être une double liaison carbone-carbone ou une triple liaison carbone-carbone, de préférence une double liaison carbone-carbone.

Par « chaîne de polymère initial », on entend au sens de la présente invention la chaîne du polymère avant la réaction de greffage ; cette chaîne comprenant au moins une insaturation susceptible de réagir avec le composé de formule (I) décrit ci-dessus. Le polymère initial est donc le polymère servant de réactif de départ lors de la réaction de greffage. La réaction de greffage permet à partir d'un polymère initial d'obtenir un polymère modifié.

De préférence, ce polymère initial est un élastomère, plus préférentiellement encore un élastomère diénique.

Par élastomère (ou indistinctement caoutchouc) « diénique », qu'il soit naturel ou synthétique, doit être compris de manière connue un élastomère constitué au moins en partie (i.e., un homopolymère ou un copolymère) d'unités monomères diènes (monomères porteurs de deux doubles liaisons carbone-carbone, conjuguées ou non).

Ces élastomères diéniques peuvent être classés dans deux catégories : « essentiellement insaturés » ou « essentiellement saturés ». On entend en général par « essentiellement insaturé », un élastomère diénique issu au moins en partie de monomères diènes conjugués, ayant un taux de motifs ou unités d'origine diénique (diènes conjugués) qui est supérieur à 15 % (% en moles); c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques « essentiellement saturés » (taux de motifs d'origine diénique faible ou très faible, toujours inférieur à 15 %).

On entend particulièrement par élastomère diénique susceptible d'être utilisé dans le cadre de la présente invention :
- tout homopolymère d'un monomère diène, conjugué ou non, ayant de 4 à 18 atomes de carbone ;
- tout copolymère d'un diène, conjugué ou non, ayant de 4 à 18 atomes de carbone et d'au moins un autre monomère.

L'autre monomère peut être l'éthylène, une oléfine ou un diène, conjugué ou non.

À titre de diènes conjugués conviennent les diènes conjugués ayant de 4 à 12 atomes de carbone, en particulier les 1,3-diènes, tels que notamment le 1,3-butadiène et l'isoprène.

À titre de diènes non conjugués conviennent les diènes non conjugués ayant de 6 à 12 atomes de carbone, tels que le 1,4-hexadiène, l'éthylidène norbornène, le dicyclopentadiène.

À titre d'oléfines conviennent les composés vinylaromatiques ayant de 8 à 20 atomes de carbone et les α-monooléfines aliphatiques ayant de 3 à 12 atomes de carbone.

À titre de composés vinylaromatiques conviennent par exemple le styrène, l'ortho-, méta-, para-méthylstyrène, le mélange commercial « vinyle-toluène », le para-tertiobutylstyrène.

À titre d'α-monooléfines aliphatiques conviennent notamment les α-monooléfines aliphatiques acycliques ayant de 3 à 18 atomes de carbone.

Plus particulièrement, l'élastomère diénique peut être :
- tout homopolymère d'un monomère diène conjugué, notamment tout homopolymère obtenu par polymérisation d'un monomère diène conjugué ayant de 4 à 12 atomes de carbone ;
- tout copolymère obtenu par copolymérisation d'un ou plusieurs diènes conjugués entre eux ou avec un ou plusieurs composés vinylaromatiques ayant de 8 à 20 atomes de carbone ;
- un copolymère d'isobutène et d'isoprène (caoutchouc butyle), ainsi que les versions halogénées, en particulier chlorées ou bromées, de ce type de copolymère ;
- tout copolymère obtenu par copolymérisation d'un ou plusieurs diènes, conjugués ou non, avec l'éthylène, une α-monooléfine ou leur mélange comme par exemple les élastomères obtenus à partir d'éthylène, de propylène avec un monomère diène non conjugué du type précité.

Préférentiellement, le polymère initial est, de préférence un élastomère diénique, choisi dans le groupe constitué par les copolymères d'éthylène-propylène-monomère diène (EPDM), le caoutchouc butyle (IRR), le caoutchouc naturel (NR), les polyisoprènes de synthèse (IR), les polybutadiènes (BR), les copolymères de butadiène, les copolymères d'isoprène et les mélanges de ces élastomères.

Préférentiellement, le polymère initial est, de préférence un élastomère diénique, choisi dans le groupe constitué par les copolymères d'éthylène-propylène-monomère diène (EPDM), le caoutchouc butyle (IRR), le caoutchouc naturel (NR), les polyisoprènes de synthèse (IR), les polybutadiènes (BR), les copolymères de butadiène-styrène (SBR), les copolymères d'éthylène-butadiène (EBR), les copolymères d'isoprène-butadiène (BIR) ou les copolymères d'isoprène-butadiène-styrène (SBIR), les copolymères d'isobutène-isoprène (caoutchouc butyle - IIR), les copolymères d'isoprène-styrène (SIR) et les mélanges de ces élastomères.

Préférentiellement, le polymère initial est, de préférence un élastomère diénique, choisi dans le groupe constitué par les copolymères d'éthylène-propylène-monomère diène, le caoutchouc butyle et le mélange de ces caoutchoucs.

Préférentiellement, le polymère initial est, de préférence un élastomère diénique, choisi dans le groupe constitué par le caoutchouc naturel, les polyisoprènes de synthèse, les polybutadiènes, les copolymères de butadiène, les copolymères d'isoprène et les mélanges de ces élastomères.

Plus préférentiellement, le polymère initial est, de préférence un élastomère diénique, choisi dans le groupe constitué par le caoutchouc naturel, les polyisoprènes de synthèse, les polybutadiènes, les copolymères de butadiène-styrène, les copolymères d'éthylène-butadiène, les copolymères d'isoprène-butadiène, les copolymères d'isoprène-butadiène-styrène, les copolymères d'isobutène-isoprène, les copolymères d'isoprène-styrène et les mélanges de ces élastomères.

Préférentiellement, le polymère initial est, de préférence un élastomère diénique, est choisi dans le groupe constitué par les polybutadiènes, les copolymères de butadiène, les copolymères d'isoprène et les mélanges de ces élastomères.

Plus préférentiellement, le polymère initial est un élastomère diénique choisi dans le groupe constitué par les polybutadiènes, les copolymères de butadiène-styrène, les copolymères d'éthylène-butadiène, les copolymères d'isoprène-butadiène, les copolymères d'isoprène-butadiène-styrène, les copolymères d'isobutène-isoprène, les copolymères d'isoprène-styrène et les mélanges de ces élastomères.

Conviennent les polybutadiènes et en particulier ceux ayant une teneur (% molaire) en unités -1,2 comprise entre 4% et 80% ou ceux ayant une teneur (% molaire) en cis-1,4 supérieure à 80%, les polyisoprènes, les copolymères de butadiène-styrène et en particulier ceux ayant une Tg (température de transition vitreuse (Tg, mesurée selon ASTM D3418 (2014)) comprise entre 0°C et - 90°C et plus particulièrement entre - 10°C et - 70°C, une teneur en styrène comprise entre 1% et 60% en poids et plus particulièrement entre 20% et 50%, une teneur (% molaire) en liaisons -1,2 de la partie butadiènique comprise entre 4% et 75%, une teneur (% molaire) en liaisons trans-1,4 comprise entre 10% et 80%, les copolymères de butadiène-isoprène et notamment ceux ayant une teneur en isoprène comprise entre 5% et 90% en poids et une Tg de - 40°C à - 80°C, les copolymères isoprène-styrène et notamment ceux ayant une teneur en styrène comprise entre 5 % et 50 % en poids et une Tg comprise entre - 5 C et - 50°C. Dans le cas des copolymères de butadiène-styrène-isoprène conviennent notamment ceux ayant une teneur en styrène comprise entre 5 % et 50 % en poids et plus particulièrement comprise entre 10 % et 40 %, une teneur en isoprène comprise entre 15 % et 60 % en poids et plus particulièrement entre 20 % et 50 %, une teneur en butadiène comprise entre 5 % et 50 % en poids et plus particulièrement comprise entre 20 % et 40 %, une teneur (% molaire) en unités -1,2 de la partie butadiènique comprise entre 4 % et 85 %, une teneur (% molaire) en unités trans -1,4 de la partie butadiènique comprise entre 6 % et 80 %, une teneur (% molaire) en unités -1,2 plus -3,4 de la partie isoprènique comprise entre 5 % et 70 % et une teneur (% molaire) en unités trans -1,4 de la partie isoprènique comprise entre 10 % et 50 %, et plus généralement tout copolymère butadiène-styrène-isoprène ayant une Tg comprise entre - 5°C et - 70°C.

Les polymères initiaux utilisables dans le cadre de l'invention, de préférence les élastomères, plus préférentiellement les élastomères diéniques, peuvent avoir toute microstructure qui est fonction des conditions de polymérisation utilisées. Ces polymères, peuvent être par exemple à blocs, statistiques, séquencés, microséquencés, et être préparer en dispersion en émulsion ou en solution. Ils peuvent être couplés et/ou étoilés, par exemple au moyen d'un atome silicium ou d'étain qui lie entre elles les chaînes polymères.

Selon l'invention, le polymère initial, de préférence l'élastomère, plus préférentiellement encore l'élastomère diénique est modifié par greffage d'un composé de formule (I) tel que défini ci-dessus aussi appelé agent de modification.

Conformément à la formule (I), cet agent de modification contient un groupement Q désignant un dipôle comprenant au moins un atome d'azote.

Par « dipôle » au sens de la présente invention, on entend une fonction capable de former une addition dipolaire 1,3 sur une liaison carbone-carbone insaturée.

De préférence, le dipôle comprenant au moins un atome d'azote est choisi parmi le groupe constitué par l'oxyde de nitrile, la nitrone et la nitrile imine.

Par oxyde de nitrile, on entend au sens de la présente invention un dipôle répondant à la formule -C≡N→O, y compris ses formes mésomères.

Par imine de nitrile, on entend au sens de la présente invention un dipôle répondant à la formule -C≡N→N, y compris ses formes mésomères.

Par nitrone, on entend au sens de la présente invention un dipôle répondant à la formule -C=N(->O)-, y compris ses formes mésomères.

Plus préférentiellement, Q est un groupement de formule (II), (III) ou (IV) 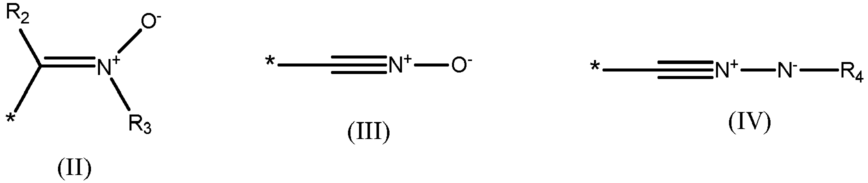 dans lesquelles :
- le symbole * représente le rattachement de Q à A ; et
- R₂ et R₄ sont choisis, indépendamment l'un de l'autre, parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ;
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

De préférence, R₂ et R₄ sont choisis indépendamment l'un de l'autre parmi un atome d'hydrogène, un alkyle en C₁-C₂₀, linéaire ou ramifié, un cycloalkyle en C₃-C₃₀ éventuellement par un ou plusieurs alkyles en C₁-C₆, linéaires ou ramifiés, un aryle en C₆-C₂₀ éventuellement substitué par un ou plusieurs alkyles en C₁-C₆, linéaires ou ramifiés et R₃ est choisi dans le groupe constitué par les alkyles en C₁-C₂₀ linéaires ou ramifiés, les cycloalkyles en C₃-C₂₀ éventuellement substitués par un ou plusieurs alkyles en C₁-C₆, linéaires ou ramifiés et les aryles en C₆-C₂₀ éventuellement substitués par un ou plusieurs alkyles en C₁-C₆, linaires ou ramifiés.

Préférentiellement, le composé de formule (I) est choisi parmi les composés de formule (IIa) 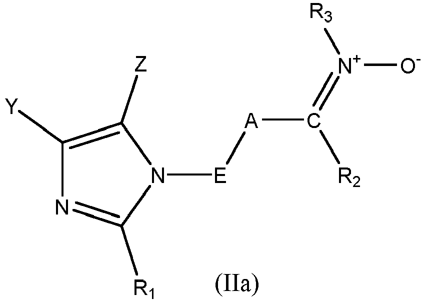 dans laquelle :
- A représente un cycle hétéroaromatique divalent éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ;
- Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
- R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

Les composés de l'invention de formule (I) et (IIa) contiennent un groupement A qui représente un cycle hétéroaromatique divalent éventuellement substitué par une ou plusieurs chaînes hydrocarbonées, identiques ou différentes, indépendantes les unes des autres, éventuellement substituées ou interrompues par un ou plusieurs hétéroatomes.

Par « cycle hétéroaromatique divalent » on entend un système aromatique cyclique comprenant un ou plusieurs hétéroatomes choisis dans le groupe constitué par l'azote, le soufre et l'oxygène. Ce système peut être monocyclique ou bicyclique et peut être formé de 5 à 10 atomes. De préférence, ce système est monocyclique et est formé de 5 à 6 atomes. Ce système peut éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes tels que par exemple O, N et S.

De préférence, lorsque le cycle hétéroaromatique divalent est substitué par une ou plusieurs chaînes hydrocarbonées, identiques ou différentes, aliphatiques, linéaires ou ramifiées, éventuellement substituées ou interrompues par un ou plusieurs hétéroatomes, cette ou ces chaînes sont de préférence inertes vis-à-vis du cycle imidazole portant les substituants R₁, X et Y et vis-à-vis du groupement Q.

On entend, au sens de la présente invention, par « chaîne(s) hydrocarbonée(s) inerte(s) vis-à-vis du cycle imidazole portant les substituants R₁, X et Y et vis-à-vis du groupement Q » une chaîne hydrocarbonée qui ne réagit ni avec ledit hétérocycle imidazole ni avec ledit groupement Q. Ainsi, ladite chaîne hydrocarbonée inerte par rapport audit hétérocycle et audit groupement est, par exemple, une chaîne hydrocarbonée qui ne présente pas de fonctions alcényle ou alcynyle, susceptibles de réagir avec ce cycle ou ce groupement. De manière préférée, ces chaînes hydrocarbonées comportent de 1 à 24 atomes de carbone et sont saturées.

De préférence, A est un cycle hétéroaromatique divalent formé de 5 à 10 atomes, de préférence de 5 à 6 atomes, éventuellement substitué par une ou plusieurs chaînes hydrocarbonées en C₁-C₂₄, identiques ou différentes, aliphatiques, de préférence saturées, linéaires ou ramifiées, éventuellement substituées ou interrompues par un ou plusieurs hétéroatomes.

Plus préférentiellement encore, dans les composés de formule (I) et (IIa), A est un cycle hétéroaromatique divalent formé de 5 à 10 atomes, de préférence de 5 à 6 atomes, éventuellement substitué par un ou plusieurs substituants choisis dans le groupe constitué par les alkyles linéaires ou ramifiés, en C₁-C₁₂, (plus préférentiellement en C₁-C₆, plus préférentiellement encore en C₁-C₄), les groupes -OR', les groupes -NHR', les groupes -SR' où R' est un groupe alkyle en C₁-C₁₂, de préférence en C₁-C₆, plus préférentiellement encore en C₁-C₄.

Préférentiellement encore, dans les composés de formule (I) et (IIa), A est choisi parmi le furane-diyle, le thiophène-diyle, le pyrrole-diyle, le thiazole-diyle, l'imidazole-diyle, la pyridine-diyle, la pyrazine-diyle, la pyrimidine-diyle, l'indole-diyle, le benzofurane-diyle, l'isoindole-diyle, l'isobenzofurane-diyle et le benzothiophène-diyle ; ces cycles étant éventuellement substitués par une ou plusieurs chaînes hydrocarbonées, identiques ou différentes, aliphatiques, de préférence saturées, éventuellement substituées ou interrompues par un ou plusieurs hétéroatomes tel que par exemple O, N et S; plus préférentiellement ces cycles pouvant être substitués par un ou plusieurs alkyles en C₁-C₆. Plus préférentiellement encore, ces cycles ne sont pas substitués.

Plus préférentiellement, dans les composés de formule (I) et (IIa), A est choisi parmi furane-diyle, le thiophène-diyle, le pyrrole-diyle, plus préférentiellement encore est le furane-diyle.

Parmi les composés de formule (IIa), ceux de la formule (IIb) sont particulièrement préférés 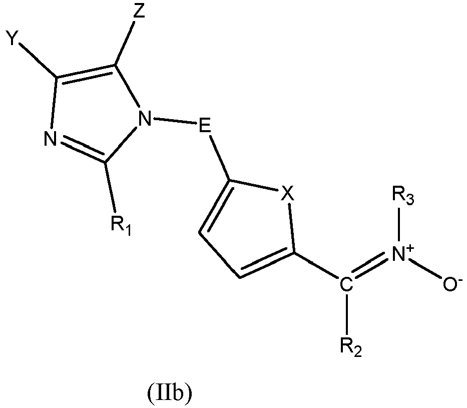 dans laquelle :
- X représente un hétéroatome choisi parmi l'atome de soufre, l'atome d'oxygène ou l'atome d'azote, de préférence X est un atome d'oxygène ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ;
- Y et Z, identiques ou différentes, représentent chacun un atome d'hydrogène ou un groupe hydrocarboné, Y et Z pouvant également former ensemble un cycle, notamment aromatique, avec les atomes de carbone du cycle imidazole auxquels ils se rattachent ;
- R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

Les composés de l'invention de formule (I), (IIa) et (IIb) contiennent un groupement E qui représente un groupe de liaison divalent hydrocarboné pouvant éventuellement contenir un ou plusieurs hétéroatomes. Par « groupe de liaison divalent hydrocarboné » on entend au sens de la présente invention, un groupe espaceur formant un pont entre le groupement A et le cycle imidazole portant les substituants R₁, X et Y, ce groupe espaceur étant une chaîne hydrocarbonée, aliphatique, de préférence saturée, linéaire ou ramifiée, de préférence en C₁-C₂₄, pouvant éventuellement contenir un ou plusieurs hétéroatome(s) tel(s) que par exemple N, O et S. Ladite chaîne hydrocarbonée peut éventuellement être substituée, pour autant que les substituants ne réagissent pas avec le groupement Q et le cycle imidazole portant les substituants R₁, X et Y.

Préférentiellement, dans les composés de l'invention de formule (I), (IIa) et (IIb), E est choisi parmi les chaînes hydrocarbonées, aliphatiques, saturées, linéaires ou ramifiées, en C₁-C₂₄, plus préférentiellement en C₁-C₁₀, encore plus préférentiellement en C₁-C₆, éventuellement interrompues par un ou plusieurs hétéroatome(s), tel(s) que N, S et O.

De préférence, dans les composés de l'invention de formule (I), (IIa) et (IIb), E est choisi dans le groupe constitué par -R-, -NH-R-, -O-R- et -S-R- avec R un alkylène linéaire ou ramifié en C₁-C₂₄, de préférence en C₁-C₁₀, plus préférentiellement en C₁-C₆.

Plus préférentiellement encore, dans les composés de l'invention de formule (I), (IIa) et (IIb), E est choisi dans le groupe constitué par -R- et -O-R- avec R un alkylène linéaire ou ramifié en C₁-C₂₄, de préférence en C₁-C₁₀, plus préférentiellement en C₁-C₆.

Plus préférentiellement encore, dans les composés de l'invention de formule (I), (IIa) et (IIb), E est choisi parmi -CH₂-, -CH₂-CH₂-, -CH₂-CH₂-CH₂-, -CH₂-CH₂-CH₂-CH₂-, -O-CH₂-, -O-CH₂-CH₂-, -O-CH₂-CH₂-CH₂- et -O-CH₂-CH₂-CH₂- CH₂-.

Plus préférentiellement encore, dans les composés de l'invention de formule (I), (IIa) et (IIb), E est choisi parmi un alkylène linéaire ou ramifié en C₁-C₂₄, de préférence en C₁-C₁₀, plus préférentiellement en C₁-C₆, plus préférentiellement encore est choisi parmi -CH₂-, -CH₂-CH₂-, -CH₂-CH₂-CH₂-, -CH₂-CH₂-CH₂-CH₂-.

Plus préférentiellement encore, dans les composés de formule (I) et (IIa), E est choisi parmi -CH₂-, -CH₂-CH₂-, -CH₂-CH₂-CH₂- et -CH₂-CH₂-CH₂-CH₂-.

Les composés de l'invention de formule (I), (IIa) et (IIb) contiennent un groupe R₁ qui représente un atome d'hydrogène ou un groupe alkyle en C₁-C₂₀. Préférentiellement, dans les composés de l'invention de formule (I), (IIa) et (IIb), R₁ représente un atome d'hydrogène ou un groupe alkyle en C₁-C₆, de préférence le méthyle. Ces groupes alkyles peuvent être linéaires ou ramifié.

Les composés de l'invention de formule (I), (IIa) et (IIb) contiennent des groupes Y et Z, identiques ou différentes, qui représentent chacun un atome d'hydrogène ou une chaîne hydrocarbonée, Y et Z pouvant également former ensemble un cycle, notamment aromatique, avec les atomes de carbone du cycle imidazole auxquels ils se rattachent.

Préférentiellement, dans les composés de l'invention de formule (I), (IIa) et (IIb), Y et Z, identiques ou différentes, représentent chacun un atome d'hydrogène ou un alkyle, linéaire ou ramifiée, en C₁-C₂₄, de préférence en C₁-C₁₀, plus préférentiellement en C₁-C₆, Y et Z pouvant également former ensemble un cycle, notamment aromatique, avec les atomes de carbone du cycle imidazole auxquels ils se rattachent.

Selon un mode de réalisation préféré, dans les composés de l'invention de formule (I), (IIa) et (IIb), Y et Z sont un atome d'hydrogène.

Selon un autre mode réalisation préféré, dans les composés de l'invention de formule (I), (IIa) et (IIb), Y et Z forment ensemble un cycle aromatique avec les atomes de carbone du cycle imidazole auxquels ils se rattachent, de préférence Y et Z forment un cycle benzénique avec les atomes de carbone du cycle imidazole auxquels ils se rattachent.

Préférentiellement, dans les composés de formule (IIa) et (IIb), R₂ représente un atome d'hydrogène ou un groupe choisi parmi les alkyles en C₁-C₂₀ linéaires ou ramifiés, les cycloalkyles en C₃-C₂₀ éventuellement substitués par un alkyle en C₁-C₆ ou les aryles en C₆-C₂₀ éventuellement substitués un alkyle en C₁-C₆.

Préférentiellement, dans les composés de formule (IIa) et (IIb), R₃ est choisi parmi les alkyles en C₁-C₂₀ linéaires ou ramifiés, les cycloalkyles en C₃-C₂₀ éventuellement substitués par un alkyle en C₁-C₆ ou les aryles en C₆-C₂₀ éventuellement substitués un alkyle en C₁-C₆.

Plus préférentiellement encore, dans les composés de formule (IIa) et (IIb), R₂ est un atome d'hydrogène et R₃ est choisi parmi les alkyles en C₁-C₂₀ et les aryles en C₆-C₂₀.

Plus préférentiellement encore, dans les composés de formule (IIa) et (IIb), R₂ est un atome d'hydrogène et R₃ est le phényle.

Parmi les composés de formule (I), (IIa) et (IIb), le composé de formule (VIII) est particulièrement préféré

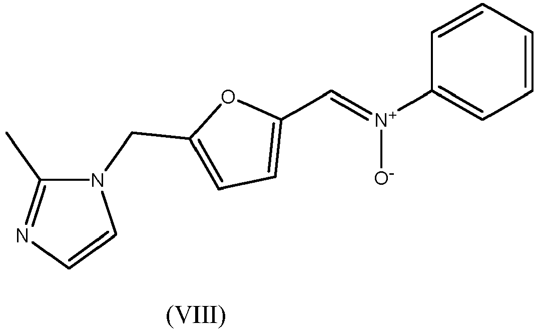

De manière surprenante, le composé de formule (I), de préférence le composé de formule (IIa) ou (IIb), plus préférentiellement encore le composé de formule (VII) permet d'obtenir des polymères modifiés par greffage et portant des groupes pendants fonctionnels imidazole plus rapidement et avec un meilleur rendement que les composés de l'art antérieur.

Les agents de modification de formule (IIa), ainsi que leurs modes de réalisation préférées peuvent être obtenus, par exemple, à partir d'un procédé de préparation d'un composé (IIa) tel que défini ci-dessus, ledit procédé comprenant au moins une réaction (c) d'un composé de formule (III) avec un composé de formule (IV) selon le schéma réactionnel suivant 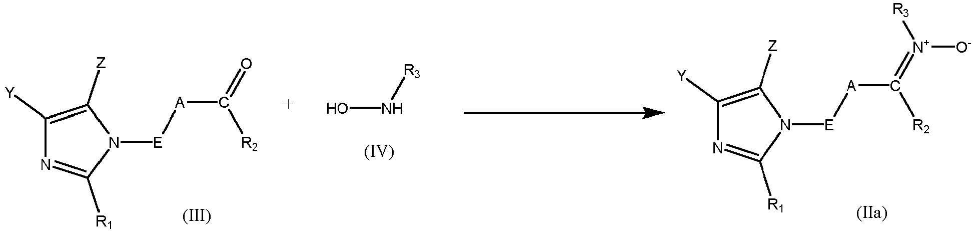 avec dans les formules (IIa), (III) et (IV) :
- A représente un cycle hétéroaromatique divalent éventuellement substitué par une ou plusieurs chaînes hydrocarbonées, identiques ou différentes, aliphatiques, de préférence saturées, linéaires ou ramifiées, éventuellement substituées ou interrompues par un ou plusieurs hétéroatomes ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ;
- Y et Z, identiques ou différentes, représentent chacun un atome d'hydrogène ou un groupe hydrocarboné, Y et Z pouvant également former ensemble un cycle, notamment aromatique, avec les atomes de carbone du cycle imidazole auxquels ils se rattachent ;
- R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

Les modes préférés de A, E, R₁, R₂, R₃ Y et Z s'appliquent également au procédé de préparation d'un composé de formule (IIa) à partir d'un composé de formule (III) et de formule (IV).

L'homme du métier sait adapter la réaction décrite ci-dessus pour obtenir les composés de formule (I) selon l'invention à partir du composé de formule (III).

Le composé de formule (IV) est disponible commercialement auprès de fournisseurs tels que Sigma Aldrich, Fischer...

Le procédé de préparation du composé de préparation d'un composé (IIa) tel que défini ci-dessus, comprend en outre au moins une réaction (b) d'un composé de formule (V) avec au moins un composé de formule (IV) pour former le composé de formule (III) 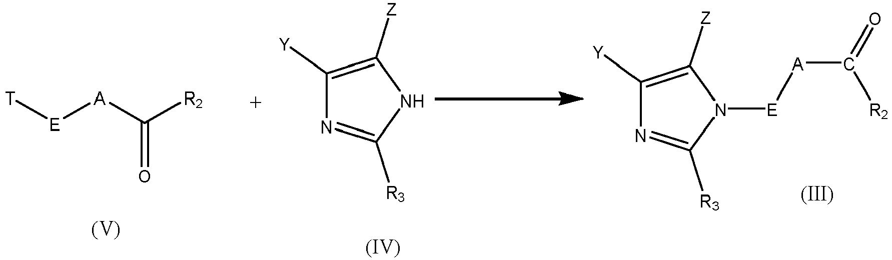 avec :
- les groupes E, A, R₁, R₂, R₃, Y et Z sont tels que définis précédemment, y compris leurs modes préférés, et
- le groupe T est choisi parmi le chlore, le brome, l'iode, le fluor, le groupe mésylate, le groupe tosylate, le groupe acétate, et le groupe trifluorométhylsulfonate.

Le composé de formule (IV) est disponible commercialement auprès des fournisseurs de produits chimiques tels que Aldrich, ABCR, ...

Le procédé de préparation du composé de préparation d'un composé (IIa) tel que défini ci-dessus, comprend en outre au moins une réaction (a) d'activation électrophile du composé de formule (VII) pour former le composé de formule (V) en présence d'une agent activation électrophile selon le schéma réactionnel suivant : 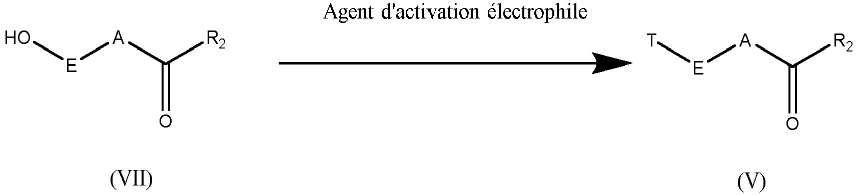 avec
- les groupes E, A et R₂ tels qu'ils sont définis ci-dessus, y compris leurs modes préférés, T un groupe partant apporté par l'agent d'activation électrophile.
- le groupe T représente est choisi parmi le chlore, le brome, l'iode, le fluor, le groupe mésylate, le groupe tosylate, le groupe acétate, et le groupe trifluorométhylsulfonate.

Par « agent d'activation électrophile », on entend un agent qui réagissant avec un groupe hydroxyle -OH lui confère un caractère électrophile. Ces agents d'activations électrophiles et cette réaction d'activation électrophile sur un groupe hydroxyle sont bien connus de l'homme du métier. On peut citer, à titre d'agent d'activation électrophile par exemple, le chlorure de thionyle, le chlorure de mésyle, le chlorure de 4-toluènesulfonyle, le chlorure d'acide paratoluène sulfonique etc.

Préférentiellement, le groupe T partant apporté par l'agent électrophile est choisi dans le groupe constitué par le chlore, le brome, l'iode, le groupe mésylate, le groupe tosylate.

Les composés de formules (VII) et (VI) sont disponibles commercialement auprès des fournisseurs de produits chimiques tels que Aldrich, ABCR, ...

Selon un mode de réalisation préférentiel, le procédé de préparation d'un composé de formule (IIa) comprend au moins les réactions successives suivantes : la réaction (b) suivit de la réaction (c) telles qu'elles ont été définies précédemment.

Selon un autre mode de réalisation préférentiel, le procédé de préparation d'un composé de formule (IIa) comprend au moins les réactions successives suivantes : la réaction (a), suivi de la réaction (b), suivi de la réaction (c) telles qu'elles ont été définies précédemment.

Selon un autre mode de réalisation préférentiel, le procédé de préparation d'un composé de formule (IIa) comprend au moins les réactions successives suivantes : la réaction (a), suivi de la réaction (b), suivi de la réaction (c) telles qu'elles ont été définies précédemment. Préférentiellement dans ce mode de réalisation, le composé de formule (VII) est obtenu par déshydratation du fructose ou du glucose ; plus préférentiellement encore le composé de formule (VII) est obtenu par déshydratation du fructose biosourcé ou du glucose biosourcé. Par « fructose biosourcé » et « glucose biosourcé » on entend au sens de la présente invention du fructose et du glucose issu de la biomasse qui peuvent être différenciés respectivement du fructose et du glucose synthétisés à partir de matières premières fossiles par les méthodes décrites dans la norme ASTM D6866-12.

Comme expliqué précédemment, les composés de formule (I), en particulier ceux de formule (IIa), plus particulièrement ceux de formules (IIb), et encore plus particulièrement le composé de formule (VII), sont utilisés comme agent de modification d'un polymère. Ils peuvent être greffés sur un ou plusieurs polymères comprenant au moins une liaison carbone-carbone insaturée, en particulier ce polymère peut être un élastomère et plus particulièrement un élastomère diénique tels que définis précédemment. Les composés de formule (I), en particulier ceux de formule (IIa) ou (IIb), en particulier ceux de formule (VII) présentent avantageusement une cinétique de greffage améliorées sur des polymères par rapport aux composés de l'art antérieur.

L'invention a également pour objet un procédé de préparation d'un polymère modifié, ledit procédé comprenant une étape de greffage sur un polymère initial comprenant au moins une insaturation d'un composé de formule (I), en particulier le composé de formule (IIa), plus particulièrement le composé de formule (IIb), plus particulièrement encore celui de formule (VII), tels que définis-ci-dessus, y compris leurs modes de réalisation préférés, par cycloaddition [3+2] du groupement Q du composé de formule (I), respectivement de la nitrone du composé de formule (IIa) ou du composé de formule (IIb), plus particulièrement encore celle de formule (VII) sur ladite insaturation.

Le greffage de ces composés est effectué par cycloaddition [3+2] du groupe Q (respectivement de la nitrone) desdits composés sur une liaison carbone-carbone insaturée de la chaîne du polymère. Le mécanisme de cette cycloaddition est notamment illustré dans le document WO2012/007441. Lors de cette réaction, ledit composé de formule (I), en particulier celui de formule (IIa) ou le composé de formule (IIb), plus particulièrement encore celui de formule (VII), forme des liaisons covalentes avec la chaîne du polymère.

Selon l'invention, le polymère porte le long de la chaîne polymère principale un ou plusieurs groupes pendants issus de la réaction de greffage des composés de formules (I), en particulier le composé de formule (IIa), plus particulièrement le composé de formule (IIb), plus particulièrement encore celui de formule (VII), tels que définis ci-dessus. Avantageusement, ces groupes pendants sont répartis le long de la chaîne polymère principale de façon aléatoire.

Selon un mode de réalisation préféré, le taux molaire de greffage du composé de formule (I), en particulier le composé de formule (IIa), plus particulièrement le composé de formule (IIb), plus particulièrement encore celui de formule (VII), est compris dans un domaine allant de 0,01 % à 15 %, de préférence de 0,05 % à 10 %, plus préférentiellement de 0,07 à 5 %.

Par « taux molaire de greffage » on entend le nombre de mol de composé de formule (I), en particulier le composé de formule (IIa), plus particulièrement le composé de formule (IIb), plus particulièrement encore celui de formule (VII), greffé sur le polymère pour 100 moles d'unité monomère constituant le polymère initial. Le taux molaire de greffage peut être déterminé par les méthodes conventionnelles d'analyses des polymères, telles que par exemple l'analyse RMN ¹H.

Selon un mode de réalisation du procédé de préparation d'un polymère modifié, le greffage du composé de formule (I), en particulier du composé de formule (IIa), plus particulièrement le composé de formule (IIb), plus particulièrement encore celui de formule (VII), peut être réalisée en masse, par exemple dans une extrudeuse, un mélangeur interne ou dans un mélangeur externe tel qu'un mélangeur à cylindres, ou en solution.

Selon un autre mode de réalisation, le procédé de préparation d'un polymère modifié peut être effectué en solution en continu ou en discontinu. Le polymère ainsi obtenu par greffage peut être séparé de sa solution par tout type de moyen connu par l'homme du métier et en particulier par une opération de stripping à la vapeur d'eau.

De préférence, dans le procédé de l'invention, le polymère initial est un élastomère, plus préférentiellement encore est un élastomère diénique.

L'invention a également pour objet une composition comprenant au moins un polymère modifié obtenu par greffage du composé de formule (I), de préférence de formule (Ia) ou (Ib), plus préférentiellement encore de formule (VIII) tel que décrit ci-dessus et au moins un additif.

Les additifs utilisables dans la composition selon l'invention peuvent être des plastifiants (tels que des huiles plastifiantes et/ou des résines plastifiantes), des charges (renforçantes ou non renforçantes) des pigments, des agents de protection tels que cires anti-ozone, anti-ozonants chimiques, anti-oxydants, des agents anti-fatigue, des résines renforçantes (telles que décrites par exemple dans la demande WO 02/10269), un système de réticulation, par exemple à base de soufre et autres agents de vulcanisation, et/ou de peroxyde et/ou de bismaléimide. De préférence, cet additif est une charge renforçante, plus préférentiellement cet additif est une charge renforçante inorganique, plus préférentiellement encore cet additif est une silice.

En plus, des objets décrits précédemment, l'invention concerne au moins l'un des objets décrits aux réalisations suivantes :
1. Polymère modifié obtenu par greffage d'au moins un composé de formule (I) sur au moins une insaturation de la chaîne d'un polymère initial 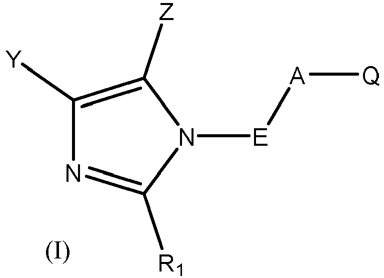 dans laquelle :
   - Q représente un dipôle comprenant au moins un atome d'azote ;
   - A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
   - E représente un groupe divalent hydrocarboné pouvant éventuellement contenir un ou plusieurs hétéroatomes ;
   - R₁ représente un atome d'hydrogène ou un groupe alkyle en C₁-C₂₀ ; et
   - Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent.
2. Polymère modifié la réalisation 2, dans lequel le taux molaire de greffage du composé de formule (I) est compris dans un domaine allant de 0,01 à 15%, de préférence de 0,05 à 10%, plus préférentiellement de 0,07 à 5%.
3. Polymère modifié selon l'une quelconque des réalisations 1 à 2, dans lequel le groupement Q est un groupe de formule (II), (III) ou (IV) 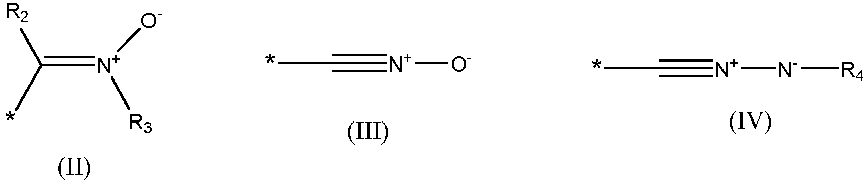 dans lesquelles :
   - le symbole * représente le rattachement de Q à A ;
   - R₂ et R₄ sont choisis, indépendamment l'un de l'autre, parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
   - R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.
4. Polymère modifié selon la réalisation 3, dans lequel le composé de formule (I) est choisi parmi les composés de formule (IIa) 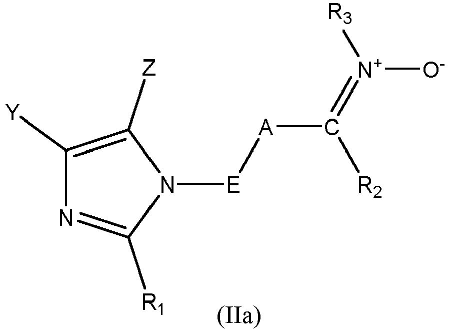 dans laquelle :
   - A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
   - E représente un groupe divalent hydrocarboné pouvant éventuellement contenir un ou plusieurs hétéroatomes ;
   - R₁ représente un atome d'hydrogène ou un groupe alkyle en C₁-C₂₀ ;
   - Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
   - R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
   - R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.
5. Polymère modifié selon l'une quelconque des réalisations 1 à 4, dans le groupement A est un cycle hétéroaromatique divalent formé de 5 à 10 atomes, de préférence de 5 à 6 atomes, éventuellement substitué par une ou plusieurs chaînes hydrocarbonées en C₁-C₂₄, identiques ou différentes, aliphatiques, linéaires ou ramifiées, éventuellement substituées ou interrompues par un ou plusieurs hétéroatomes.
6. Polymère modifié selon l'un quelconque des réalisations 1 à 5, dans lequel le groupement A est choisi parmi le furane-diyle, le thiophène-diyle, le pyrrole-diyle, le thiazole-diyle, l'imidazole-diyle, la pyridine-diyle, la pyrazine-diyle, la pyrimidine-diyle, l'indole-diyle, le benzofurane-diyle, l'isoindole-diyle, l'isobenzofurane-diyle et le benzothiophène-diyle.
7. Polymère modifié selon la réalisation 4, dans lequel le composé de formule (IIa) est choisi parmi ceux de la formule (IIb) 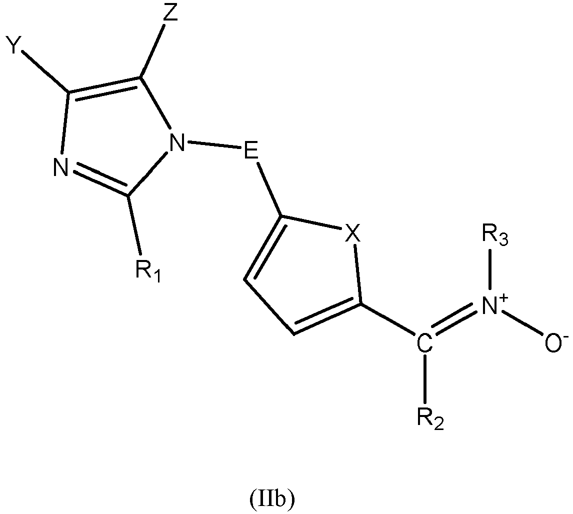 dans laquelle :
   - X représente un groupe hétéroatome choisi parmi l'atome de soufre, l'atome d'oxygène ou l'atome d'azote, de préférence X est un atome d'oxygène ;
   - E représente un groupe divalent hydrocarboné pouvant éventuellement contenir un ou plusieurs hétéroatomes ;
   - R₁ représente un atome d'hydrogène ou un groupe alkyle en C₁-C₂₀ ;
   - Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
   - R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
   - R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.
8. Polymère modifié selon l'un quelconque des réalisations 3 à 7, dans lequel R₂ est un atome d'hydrogène et R₃ est choisi parmi les alkyles en C₁-C₂₀ et les aryles en C₆-C₂₀.
9. Polymère modifié selon l'un quelconque des réalisations précédentes, dans lequel groupement E est choisi parmi les chaînes hydrocarbonées aliphatiques, linéaires ou ramifiées, saturées en C₁-C₂₄, préférentiellement en C₁-C₁₀, plus préférentiellement en C₁-C₆ éventuellement interrompues par un ou plusieurs atomes d'azote, de soufre ou d'oxygène.
10. Polymère modifié selon l'une quelconque des réalisations précédentes, dans lequel le groupement E est choisi parmi les groupes -R-, -NHR-, -OR- et -SR- où R est un alkylène, linéaire ou ramifié, en C₁-C₂₄, de préférence en C₁-C₁₀, plus préférentiellement en C₁-C₆.
11. Polymère modifié selon l'un quelconque des réalisations précédentes, dans lequel groupement E est choisi parmi les groupes -R- ou -OR- où R est un alkylène linéaire ou ramifié en C₁-C₂₄, de préférence en C₁-C₁₀, plus préférentiellement en C₁-C₆.
12. Polymère modifié selon l'un quelconque des réalisations précédentes, dans lequel groupement E est un alkylène linéaire ou ramifié en C₁-C₂₄, de préférence en C₁-C₁₀, plus préférentiellement en C₁-C₆, plus préférentiellement encore choisi parmi -CH₂-, -CH₂-CH₂-, -CH₂-CH₂-CH₂-, -CH₂-CH₂-CH₂-CH₂-.
13. Polymère modifié selon l'un quelconque des réalisations précédentes, dans lequel les groupes Y et Z, identiques ou différentes, représentent chacun un atome d'hydrogène ou un alkyle en C₁-C₂₄, de préférence en C₁-C₁₀, plus préférentiellement en C₁-C₆, Y et Z pouvant également former ensemble un cycle, notamment aromatique, avec les atomes de carbone du cycle imidazole auxquels ils se rattachent.
14. Polymère modifié selon l'un quelconque des réalisations précédentes, dans lequel Y et Z sont un atome d'hydrogène.
15. Polymère modifié selon l'un quelconque des réalisations 1 à 13, dans lequel les groupes Y et Z forment ensemble un cycle aromatique avec les atomes de carbone du cycle imidazole auxquels ils se rattachent, de préférence les groupes Y et Z forment un cycle benzénique avec les atomes de carbone du cycle imidazole auxquels ils se rattachent.
16. Polymère modifié selon l'un quelconque des réalisations précédentes, dans lequel R₁ est un atome d'hydrogène ou un alkyle en C₁-C₆, de préférence le méthyle.
17. Polymère modifié selon l'un quelconque des réalisations précédentes, caractérisé en ce qu'il est le composé de formule (VIII) 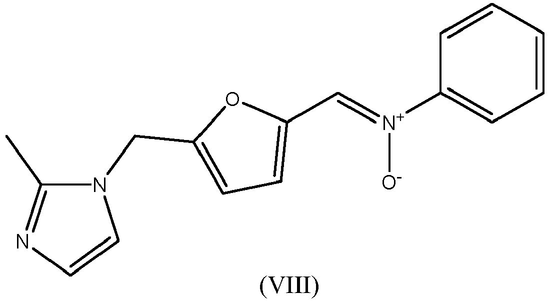
18. Polymère modifié selon l'un quelconque des réalisations précédentes, dans lequel le polymère initial est un élastomère, de préférence un élastomère diénique.
19. Polymère modifié selon la réalisation 18, dans lequel l'élastomère diénique est choisi dans le groupe constitué par les copolymères d'éthylène-propylène-monomère diène, le caoutchouc butyle, le caoutchouc naturel, les polyisoprènes de synthèse, les polybutadiènes, les copolymères de butadiène, les copolymères d'isoprène et les mélanges de ces élastomères.
20. Procédé de préparation d'un polymère modifié selon l'une quelconque des réalisations précédentes, ledit procédé comprenant une étape de greffage sur un polymère initial comprenant au moins une insaturation d'un composé de formule (I) par cycloaddition [3+2] de la fonction Q du composé de formule (I) sur ladite insaturation 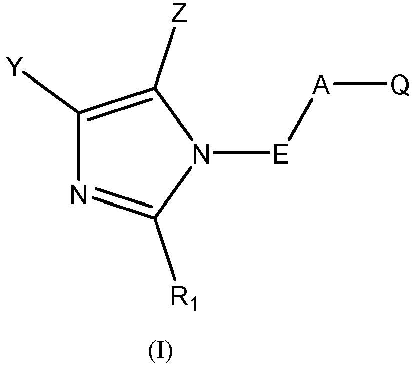 dans laquelle :
   - Q représente un dipôle comprenant au moins un atome d'azote ;
   - A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
   - E représente un groupe divalent hydrocarboné pouvant éventuellement contenir un ou plusieurs hétéroatomes ;
   - R₁ représente un atome d'hydrogène ou un groupe alkyle en C₁-C₂₀ ; et
   - Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent.
21. Procédé de préparation d'un polymère modifié selon la réalisation 20, dans lequel le composé de formule (I) est choisi parmi les composés de formule (IIa) 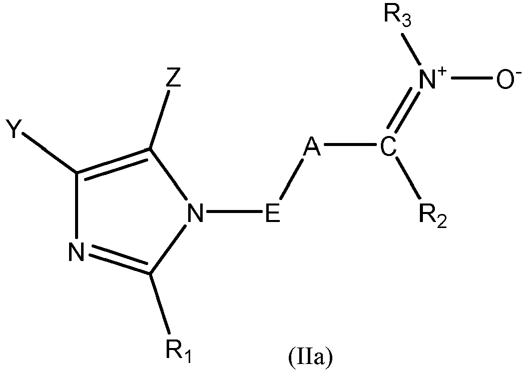 dans laquelle :
   - A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
   - E représente un groupe divalent hydrocarboné pouvant éventuellement contenir un ou plusieurs hétéroatomes ;
   - R₁ représente un atome d'hydrogène ou un groupe alkyle en C₁-C₂₀ ;
   - Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
   - R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
   - R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.
22. Procédé de préparation d'un polymère modifié selon l'une des réalisations 20 ou 21, dans lequel A est un cycle hétéroaromatique divalent formé de 5 à 10 atomes, de préférence de 5 à 6 atomes, éventuellement substitué par une ou plusieurs chaînes hydrocarbonées en C₁-C₂₄, identiques ou différentes, aliphatiques, linéaires ou ramifiées, éventuellement substituées ou interrompues par un ou plusieurs hétéroatomes.
23. Procédé de préparation d'un polymère modifié selon l'une des réalisations 20 à 22, dans lequel le composé de formule (IIa) est choisi parmi ceux de la formule (IIb) 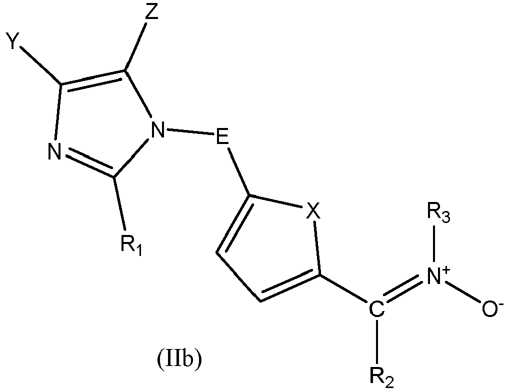 dans laquelle :
   - X représente un groupe hétéroatome choisi parmi l'atome de soufre, l'atome d'oxygène ou l'atome d'azote, de préférence X est un atome d'oxygène;
   - E représente un groupe divalent hydrocarboné pouvant éventuellement contenir un ou plusieurs hétéroatomes ;
   - R₁ représente un atome d'hydrogène ou un groupe alkyle en C₁-C₂₀ ;
   - Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
   - R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
   - R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.
24. Procédé de préparation d'un polymère modifié selon l'une quelconque des réalisations 20 à 23, caractérisé en ce que le composé de formule (I) est le composé de formule (VIII) 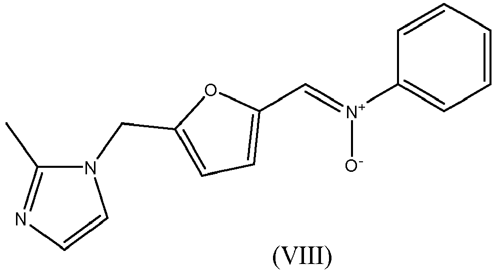
25. Composition comprenant au moins un polymère modifié selon l'une quelconque des réalisations précédentes 1 à 19 et au moins un additif, de préférence l'additif est une charge, plus préférentiellement encore l'additif est une charge renforçante, encore plus préférentiellement l'additif est une charge renforçante inorganique, plus préférentiellement l'additif est une silice.
26. Bande de roulement comprenant au moins une composition telle que définie à la réalisation 25.
27. Pneumatique comprenant au moins une composition telle que définie à la réalisation 25.
28. Pneumatique comprenant au moins une bande de roulement telle que définie à la réalisation 26.

### V-EXEMPLES

Les exemples qui suivent permettent d'illustrer l'invention, cette dernière ne saurait cependant être limitée à ces seuls exemples.

### 5.1 Caractérisations des molécules

L'analyse structurale ainsi que la détermination des puretés molaires des molécules de synthèse sont réalisées par une analyse RMN. Les spectres sont acquis sur un spectromètre Avance 3 400 MHz BRUKER équipé d'une sonde « large bande BBFO-zgrad 5 mm ». L'expérience RMN ¹H quantitative, utilise une séquence simple impulsion 30° et un délai de répétition de 3 secondes entre chacune des 64 acquisitions. Les échantillons sont solubilisés dans un solvant deutéré, le diméthylsulfoxide deutéré (DMSO) sauf indication contraire. Le solvant deutéré est également utilisé pour le signal de « lock ». Par exemple, la calibration est réalisée sur le signal des protons du DMSO deutéré à 2,44 ppm par rapport à une référence TMS à 0 ppm. Le spectre RMN ¹H couplé aux expériences 2D HSQC ¹H/¹³C et HMBC ¹H/¹³C permettent la détermination structurale des molécules (cf tableaux d'attribution). Les quantifications molaires sont réalisées à partir du spectre RMN 1D ¹H quantitatif.

### 5.2 Caractérisation des molécules greffées sur un élastomère diénique

La détermination du taux molaire du composé greffé testé sur un élastomère diénique est réalisée par une analyse RMN. Les spectres sont acquis sur un spectromètre 500 MHz BRUKER équipé d'une sonde « CryoSonde BBFO-zgrad-5 mm ». L'expérience RMN ¹H quantitative, utilise une séquence simple impulsion 30° et un délai de répétition de 5 secondes entre chaque acquisition. Les échantillons sont solubilisés dans un solvant deutéré, le chloroforme deutéré (CDCl₃) sauf indication contraire dans le but d'obtenir un signal de « lock ». Des expériences RMN 2D ont permis de vérifier la nature du motif greffé grâce aux déplacements chimiques des atomes de carbone et de proton.

### 5.3 Mesure des masses molaires moyennes en nombre (Mn), en poids (Mw) et de l'indice de polydispersité des élastomères diéniques.

Sauf indication expresse contraire, les masses molaires moyennes en nombre et en poids des élastomères diéniques utilisés sont mesurées par la technique de chromatographie d'exclusion stérique ou SEC (Size Exclusion Chromatography). La SEC permet de séparer les macromolécules en solution suivant leur taille à travers des colonnes remplies d'un gel poreux. Les macromolécules sont séparées suivant leur volume hydrodynamique, les plus volumineuses étant éluées en premier.

Sans être une méthode absolue, la SEC permet d'appréhender la distribution des masses molaires d'un élastomère. À partir de produits étalons commerciaux, les différentes masses molaires moyennes en nombre (Mn) et en poids (Mw) peuvent être déterminées et l'indice de polymolécularité (Ip = Mw/Mn) calculé via un étalonnage dit de MOORE.

Il n'y a pas de traitement particulier de l'échantillon de l'élastomère avant analyse. Celui-ci est simplement solubilisé à une concentration d'environ 1 g/l, dans du chloroforme ou dans le mélange suivant : tétrahydrofurane + 1 % vol. de diisopropylamine + 1 % vol. de triéthylamine + 1 % vol. d'eau distillée (% vol. =% volume). Puis, la solution est filtrée sur filtre de porosité 0,45 µm avant injection.

L'appareillage utilisé est un chromatographe « WATERS alliance ». Le solvant d'élution est le mélange suivant : tétrahydrofurane + 1 % vol. de diisopropylamine + 1 % vol. de triéthylamine ou du chloroforme selon le solvant utilisé pour la mise en solution de l'élastomère. Le débit est de 0,7 ml/min, la température du système de 35°C et la durée d'analyse de 90 min. On utilise un jeu de quatre colonnes WATERS en série, de dénominations commerciales « STYRAGEL HMW7 », « STYRAGEL HMW6E » et deux « STYRAGEL HT6E ».

Le volume injecté de la solution de l'échantillon de l'élastomère est 100 µL. Le détecteur est un réfractomètre différentiel « WATERS 2410 » de longueur d'onde 810 nm. Le logiciel d'exploitation des données chromatographiques est le système «WATERS EM POWER».

Les masses molaires moyennes calculées sont relatives à une courbe d'étalonnage réalisée à partir de polystyrènes étalons commerciaux « PSS READY CAL-KIT ».

### 5.4 Synthèse de l'oxyde 1-(5-((2-méthyl-1H-imidazole-1-yl)méthylfuran-2-yl)-N-phénylméthanimine (composé E)

L'oxyde 1-(5-((2-méthyl-1H-imidazole-1-yl)méthylfuran-2-yl)-N-phénylméthanimine peut être synthétisé selon le schéma réactionnel suivant

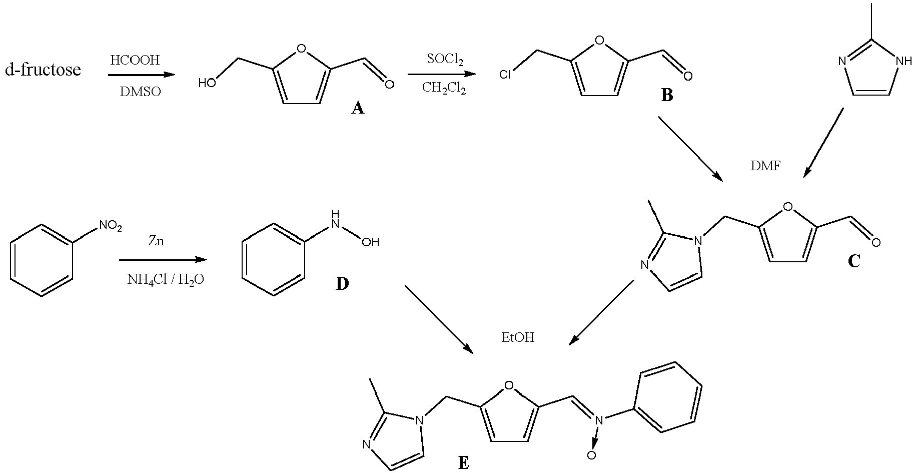

L'oxyde 1-(5-((2-méthyl-1H-imidazole-1-yl)méthylfuran-2-yl)-N-phénylméthanimine est synthétisé en 2 étapes qui sont décrites ci-après. Tous les composés chimiques utilisés lors de cette synthèse proviennent de « Sigma Aldrich » ou de « Fischer Scientific »

Le 5-(hydroxyméthyl)furan-2-carbaldéhyde (composé A, CAS 67-47-0) est commercial ou peut être synthétisé par voie biochimique ou par voie chimique à partir du fructose.

Le produit N-phénylhydroxylamine (composé D, CAS 100-65-2) est commercial ou peut-être synthétisé à partir du nitrobenzène selon la procédure décrite dans Organic Synthèses, Coll. Vol. 1. p. 445 (1941); Vol. 4. p. 57 (1925).

Le 5-(chlorométhyl)furan-2-carbaldéhyde (composé B) peut-être synthétisé à partir du fructose ou du 5-(hydroxyméthyl)furan-2-carbaldéhyde selon la procédure décrite dans Sanda, Komla et al, Synthesis of 5-(bromométhyl)- and of 5-(chlorométhyl)-2-furancarboxaldéhyde, Carbohydrate Research, 187(1), 15-23; 1989

### 5.4.1 Etape 1 : Synthèse du 5-((2-méthyl-1H-imidazol-1-yl)méthyl)furan-2-carbaldéhyde (produit C)

Un mélange de 2-méthylimidazole (4,83 g ; 58,80 mmol ; 2,5 Eq.) et de 5-(chlorométhyl)furan-2-carbaldéhyde (3,40 g ; 23,52 mmol) dans le DMF (4 ml) est chauffé jusqu'à une température de bain de 70°C. Après 2-3 heures d'agitation à cette température et 2 heures à une température de bain de 80°C, le milieu réactionnel est dilué par de l'eau (50 ml) puis la phase organique est séparée. La phase aqueuse est extraite 4 fois par du dichlorométhane (4 fois 20 ml). Les fractions de phases organiques sont réunies puis lavée par de l'eau (4 fois 5 ml) puis concentré sous pression réduite (2-3 mbar ; 32°C) pour conduire à une huile de couleur noire (2,44 g ; 12,8 mmol) avec un rendement de 55 %. Ce produit est engagé dans l'étape suivante sans purification supplémentaire.

### 5.4.2 Étape 2 : Synthèse de l'oxyde 1-(5-((2-méthyl-1H-imidazol-1-yl)méthyl)furan-2-yl)-N-phénylméthanimine (produit E)

A une solution de 5-((2-méthyl-1H-imidazol-1-yl)méthyl)furan-2-carbaldéhyde (composé C) (5,00 g ; 26,3 mmol) dans l'éthanol (5 ml) à une température de bain de 35-40°C, est ajouté la N-phénylhydroxylamine (2,87 g ; 26,3 mmol ; 1Eq.) par portions pendant 5 minutes. Le milieu réactionnel est chauffé jusqu'à une température de bain de 60°C. Après 1,5 heures d'agitation à cette température puis retour à une température de 30-35°C, est ajouté goutte à goutte du tert-butyle méthyle éther (15 ml). Après une heure d'agitation à température ambiante (23°C), le précipité obtenu est filtré et lavé sur filtre par un mélange d'éthanol et de tert-butyle méthyle éther (1 ml et 5 ml), puis par du tert-butyle méthyle éther (8 ml). Un solide clair de couleur brune et de point de fusion 147-150°C est obtenu avec un rendement de 68,4 % (5,06 g ; 17,99 mmol) et de pureté molaire supérieure à 98 % (RMN ¹H).

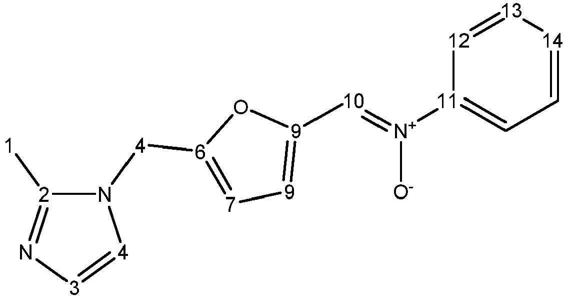

**[Table 1]**

| N° | δ ¹H (ppm) | δ ¹³C(ppm) |
|---|---|---|
| 1 | 2,37 | 12,6 |
| 2 | / | 144,2 |
| 3 | 6,87 | 127,1 |
| 4 | 6,81 | 119,0 |
| 5 | 4,99 | 42,6 |
| 6 | / | 150,7 |
| 7 | 6,39 | 110,8 |
| 8 | 7,87 | 116,4 |
| 9 | / | 147,5 |
| 10 | 8,01 | 123,1 |
| 11 | / | 146,7 |
| 12 | 7,71 | 120,5 |
| 13 | 7,41 | 128,7 |
| 14 | 7,40 | 129,6 |

### 5.5 Greffage de polymère avec le composé E

### 5.5.1 Fabrication d'un copolymère styrène-butadiène greffé par l'oxyde de 1-(5-((2-méthyl-1H-imidazol-1-yl)méthyl)furan-2-yl)-N-phénylméthanimine (composé E)

On incorpore l'oxyde de 1-(5-((2-méthyl-1H-imidazol-1-yl)méthyl)furan-2-yl)-N-phénylméthanimine (0,31; 1,14 mmol) à 15 g d'un copolymère styrène-butadiène SBR (contenant 26,5 % en poids de styrène par rapport au poids total du copolymère et 24 % en poids d'unités butadiène-1,2 par rapport au poids de la partie butadiènique, 28 % en poids d'unités butadiènique-1,4 cis par rapport au poids de la partie butadiènique et 48 % en poids d'unités butadiène-1,4 trans par rapport au poids de la partie butadiènique, Mn=120000 g/mol et Ip=1,84 mesurés selon la méthode décrite au paragraphe 5.3) sur un outil à cylindres à 23°C. Le mélange est homogénéisé en 15 passes portefeuille. Cette phase de mélangeage est suivie d'un traitement thermique à 160°C pendant 60 minutes sous presse à 10 bars de pression.

Les résultats du greffage d'après l'analyse RMN ¹H sont présentés dans le tableau 2.

### 5.5.2 Fabrication d'un polymère polybutadiène greffé par l'oxyde de 1-(5-((2-méthyl-1H-imidazol-1-yl)méthyl)furan-2-yl)-N-phénylméthanimine (composé E)

0,5 g de polybutadiène (75,4 % molaire d'unités butadiène-1,2 et 24,6 % molaire d'unités butadiène-1,4 ; de Mn=7800 g/mol et Ip=1,02 mesurés selon la méthode décrite au paragraphe 5.3) ont été balayé à l'azote pendant 15 minutes. Puis, 2 ml de dichlorométhane, préalablement barbotés à l'azote pendant 5 minutes, ont été ajoutés pour dissoudre ce polymère.

Une fois ce polymère dissous, 0,265 g d'oxyde de 1-(5-((2-méthyl-1H-imidazol-1-yl)méthyl)furan-2-yl)-N-phénylméthanimine (composé E) (0,94 mmol) préalablement dissous dans 2 ml de dichlorométhane ont été ajoutés au milieu réactionnel sous agitation. Après 15 minutes d'agitation, le milieu réactionnel a été laissé sous balayage d'azote pendant 15 minutes pour évaporer le dichlorométhane. Une fois la totalité du solvant évaporé, le milieu réactionnel a été chauffé à 150°C (température du bain), sous flux d'azote constant. Après 10h30 de réaction, le milieu réactionnel est laissé revenir à température ambiante (23°C).

Les résultats du greffage d'après l'analyse RMN ¹H sont présentés dans le tableau 2.

### 5.5.3 Fabrication d'un copolymère éthylène butadiène greffé par l'oxyde de 1-(5-((2-méthyl-1H-imidazol-1-yl)méthyl)furan-2-yl)-N-phénylméthanimine (composé E)

On incorpore l'oxyde de 1-(5-((2-méthyl-1H-imidazol-1-yl)méthyl)furan-2-yl)-N-phénylméthanimine (0,57; 2 mmol) à 15 g d'un copolymère éthylène-butadiène EBR (contenant 16,8 % molaire de butadiène, 7,7% molaire de cycle butadiène/éthylène et 75,5 % molaire d'éthylène) sur un outil à cylindres à 23°C. Le mélange est homogénéisé en 15 passes portefeuille. Cette phase de mélangeage est suivie d'un traitement thermique à 160°C pendant 60 minutes sous presse à 10 bars de pression.

Les résultats du greffage d'après l'analyse RMN ¹H sont présentés dans le tableau 2.

**[Table 2]**

| Élastomère | Taux visé (% molaire) | Composé E greffé (% molaire) | Rendement de greffage (en %) |
|---|---|---|---|
| BR | 10 | 9,3 | 93 % |
| EBR | 0,5 | 0,31 | 62 % |
| SBR | 0,47 | 0,37 | 78 % |

### 5.6. Synthèse du composé 1,3 dipolaire oxyde de la N-(4-((2-méthyle-1H-imidazol-1-yl)méthyl)benzylidène)aniline (Composé E1)

Ce composé peut être préparé en 5 étapes selon le schéma réactionnel suivant :

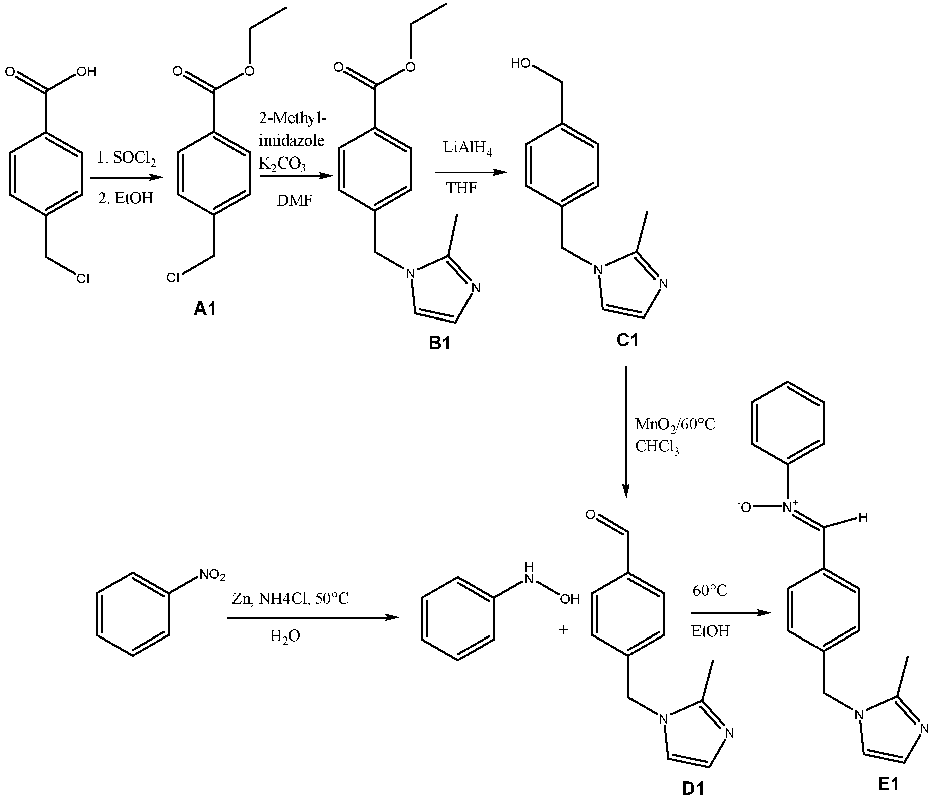

### 5.6.1 Etape 1 : Synthèse du méthyl-4-(chlorométhyl)benzoate (composé A1)

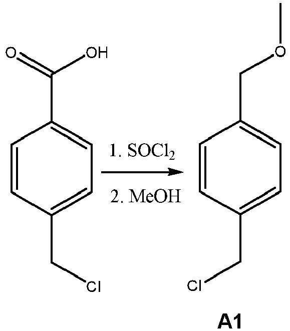

A 150 ml de méthanol refroidi jusqu'à une température -8°C (Température du bain) est ajouté le chlorure de thionyle SOCl₂ (2,4 ml ; 32,2 mmol) au goutte à goutte pendant 10 minutes à une température de -8 °C (Température du bain). Après 5 min d'agitation à -8°C (Température du bain), l'acide 4-(chlorométhyl)benzoïque (5,0 g ; 29,3 mmol) est ajouté par petits portions pendant 10 minutes à -8°C (Température du bain). Après 20 minutes d'agitation à -8°C (Température du bain), le milieu réactionnel est chauffé jusqu'à 14°C (Température du bain) pendant 20 minutes. Ensuite, le milieu réactionnel est chauffé jusqu'à 50°C (Température du bain) pendant une heure et est agité à cette température pendant 2 heures. La solution du produit est concentrée sous pression réduite (28 mbar, 40°C, Température du bain) pour conduire à une huile qui cristallise à température ambiante.

Un solide blanc (5,16 g ; 27,9 mmol ; rendement molaire de 95 %) de point de fusion 38 - 40°C est obtenu.

La pureté molaire est supérieure à 95% mol. (RMN ¹H).

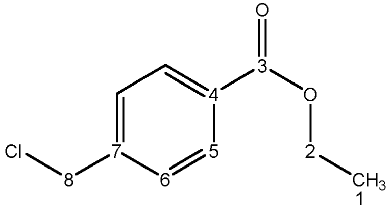

**[Table 3]**

| N° | δ ¹H (ppm) | δ ¹³C(ppm) |
|---|---|---|
| 1 | 3,92 | 45,4 |
| 2 | / | 166,5 |
| 3 | / | 128,5 |
| 4 | 8,04 | 130,0 |
| 5 | 7,47 | 129,2 |
| 6 | / | 142,2 |
| 7 | 4,62 | 52,2 |

| | | |
|---|---|---|
| Solvant: CDCl₃ | | |

### 5.6.2 Étape 2 : Synthèse du méthyl-4-((2-méthyl-1H-imidazol-1-yl))méthyl)benzoate :

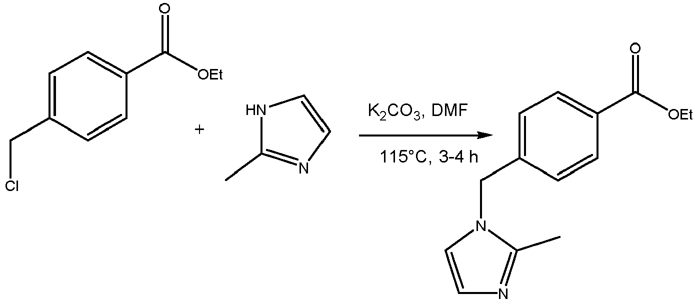

Un mélange de méthyl 4-(chlorométhyl)benzoate (5,15 g ; 28 mmol), et de 2-méthyl-1H-imidazole (2,52 g ; 31 mmol) et de K₂CO₃ (2,89 g; 21 mmol) dans DMF (4 ml) est chauffé à 60°C (Température du bain) pendant 1 -1,5 heures, puis 5 pendant heures à 80°C. Après refroidissement le milieu réactionnel est dilué par de l'eau à 0°C (50 ml) et de l'acétate d'éthyle (25 ml). La phase aqueuse est séparée est extraite par de l'acétate d'éthyle (3 fois 10 ml). Les phases organiques réunies sont lavées par de l'eau (3 fois 5 ml). La solution du produit est concentrée sous pression réduite (7 mbar, 40°C, Température du bain) pour conduire à une huile jaune (4,266 g ; 18,5 mmol, rendement molaire de 66 %).

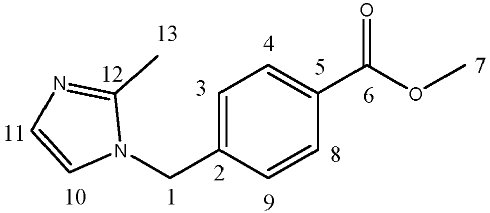

**[Table 4]**

| N° | δ ¹H (ppm) |
|---|---|
| 1 | 5,09 |
| 2 | / |
| 3 | 7,08 |
| 4 | 7,99 |
| 5 | / |
| 6 | / |
| 7 | 3,46 |
| 8 | 7,99 |
| 9 | 7,08 |
| 10 | 6,96 |
| 11 | 6,84 |
| 12 | / |
| 13 | 2,30 |

| | |
|---|---|
| Solvant: CDCl₃ | |

### 5. 6.3 Étape 3 : Synthèse du 4-((2-méthyl-1H-imidazol-1-yl))méthyl)phenylméthanol :

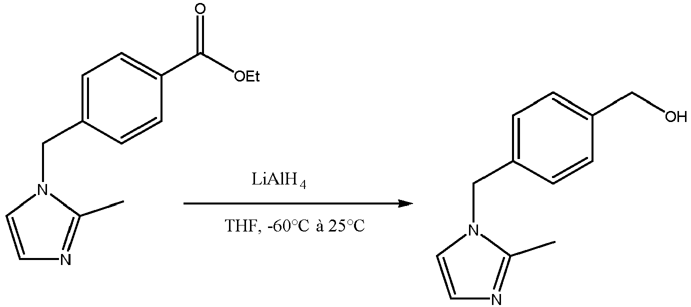

Une solution de LiAlH₄ (1,50 g, 0,039 mol) dans le THF anhydre (230 ml) est refroidi à - 60°C. Une solution d'éthyl 4-((2-méthyl-1H-imidazol-1-yl)méthyl)benzoate (7,80 g, 0,028 mol, 81% mol) dans le THF anhydre (100 ml) est ajoutée sous argon pendant 15 minutes. Le milieu réactionnel est agité pendant 1 heure à -60°C, puis 10-12 heures à température ambiante. De l'eau (20 ml) est ajoutée goutte à goutte (une réaction exothermique). Le précipité formé est filtré, Le filtrat est concentré sous pression réduite. Le brut obtenu est solubilisé dans CH₂Cl₂ (100 ml) pour faire précipiter des insolubles. Après filtration et concentration sous pression réduite, une huile jaune (4,96 g, rendement molaire 93 %) est obtenue. La pureté molaire est supérieure à 85 % (RMN ¹H).

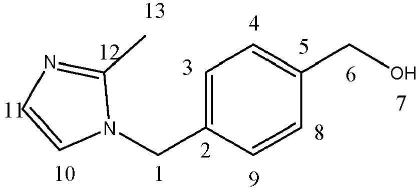

**[Table 5]**

| N° | δ ¹H (ppm) |
|---|---|
| 1 | 4,99 |
| 2 | / |
| 3 | 7,30 |
| 4 | 6,97 |
| 5 | / |
| 6 | 4,66 |
| 7 | 7,63 |
| 8 | 6,97 |
| 9 | 7,30 |
| 10 | 6,82 |
| 11 | 6,79 |
| 12 | / |
| 13 | 2,23 |

| | |
|---|---|
| Solvant: CDCl₃ | |

### 5.6.4 Étape 4 : Synthèse du 4-((2-méthyl-1H-imidazol-1-yl))méthyl)benzaldéhyde :

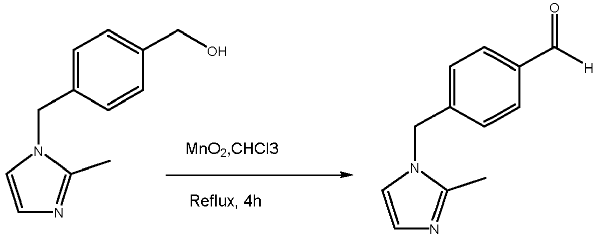

Un mélange de MnO₂ (6,88 g ; 0,079 mol) et de 4-((2-méthyl-1H-imidazol-1-yl)méthyl)phénylméthanol (4,57 g ; 0,021 mol, 85% mol. par RMN ¹H) dans CHCl₃ (180 ml) est agité pendant 4 heures à température au reflux. Le milieu réactionnel est refroidi jusqu'à température ambiante et est maintenu sous agitation à cette température pendant 10-12 heures. Les produits insolubles sont filtrés, le filtrat est concentré sous pression réduite. Une huile jaune (3,78 g, rendement molaire de 98 %) est obtenue après concentration sous pression réduite. La pureté molaire est supérieure à 81 % (RMN ¹H).

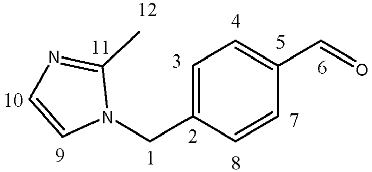

**[Table 6]**

| N° | δ ¹H (ppm) |
|---|---|
| 1 | 5,16 |
| 2 | / |
| 3 | 7,20 |
| 4 | 7,89 |
| 5 | / |
| 6 | 10,01 |
| 7 | 7,89 |
| 8 | 7,20 |
| 9 | 7,02 |
| 10 | 6,88 |
| 11 | / |
| 12 | 2,34 |

| | |
|---|---|
| Solvant: CDCl₃ | |

### 5. 6.5 Synthèse de la phénylhydroxylamine :

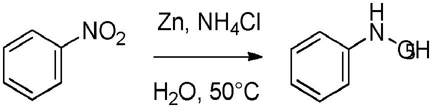

La phénylhydroxylamine a été synthétisé selon le mode opératoire décrit dans Org.Syntheses, Coll. Vol. 1 , p. 445, 1941; Org.Syntheses, Coll. Vol. 3 , p. 668, 1955

### 5.6.6 Étape 5 : Synthèse du N-4-((2-méthyl-1H-imidazol-1yl))méthyl)benzyldiène)aniline oxyde :

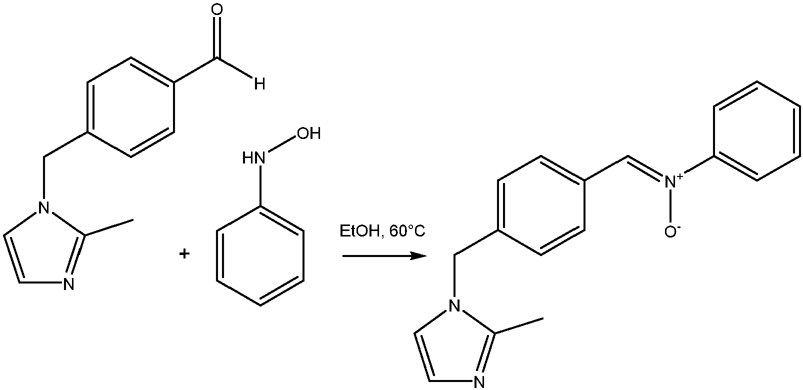

Une solution du 4-((2-méthyl-1H-imidazol-1-yl))méthyl)benzaldéhyde (3,48 g ; 0,015 mol, 81% mol. par ¹H RMN) et de phénylhydroxylamine (2,86 g ; 0,026 mol) dans l'éthanol anhydre (20 ml) est agitée pendant 2 heures à 60°C (Température du bain) et ensuite pendant 12 heures à température ambiante. On filtre le précipité jaune (0,249 g contenant le produit attendu). Au filtrat, on ajoute de l'eau (30 ml) sous vive agitation. Le précipité jaune alors formé est filtré après 20 minutes d'agitation et lavé par un mélange d'EtOH (10 ml) et d'eau (20 ml), puis par l'eau (50 ml). Les deux portions de solide sont réunies et séchées pendant 10-12 heures sous pression atmosphérique à température ambiante. Un solide jaune (3,71 g, rendement molaire 89 %) de la pureté molaire supérieure à 82 % (RMN ¹H) est obtenu. Une purification supplémentaire est appliquée par agitation pendant 1.5 heures à température ambiante, filtration, lavage sur le filtre par 50 ml d'éther éthylique, séchage pendant 2 jours à température ambiante.

Un solide jaune (3,04 g, rendement molaire 78 %) de point de fusion 115-116°C est obtenu. La pureté molaire est supérieure à 88 % (RMN ¹H).

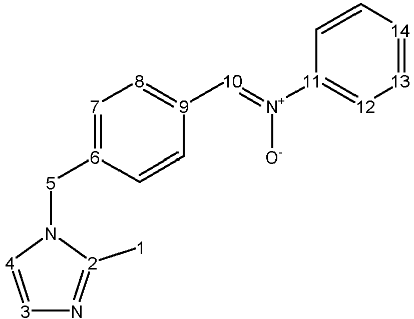

**[Table 7]**

| N° | δ ¹H (ppm) | δ ¹³C(ppm) |
|---|---|---|
| 1 | 2,28 | 13,1 |
| 2 | / | 145,0 |
| 3 | 6,93 | 127,6 |
| 4 | 6,81 | 119,9 |
| 5 | 5,05 | 49,6 |
| 6 | / | 139,0 |
| 7 | 7,11 | 126,8 |
| 8 | 8,32 | 129,5 |
| 9 | / | 130,5 |
| 10 | 7,85 | 133,6 |
| 11 | / | 149,1 |
| 12 | 7,72 | 121,7 |
| 13 | 7,43 | 129,2 |
| 14 | 7,42 | 130,1 |

| | | |
|---|---|---|
| Solvant: CDCl₃ | | |

### 5.7. Etude du rendement de greffage du composé E en fonction du temps.

Dans cet essai, on compare, sur un copolymère styrène/butadiène SBR le rendement de greffage en fonction du temps d'un composé 1,3-dipolaire selon l'invention (composé E) par rapport à celle d'un composé 1,3 dipolaire de l'art antérieur (Composé E1).

Un copolymère styrène-butadiène utilisé est un SBR contenant 26,5 % en poids de styrène par rapport au poids total du copolymère et dans sa partie butadiènique, par rapport au poids de la partie butadiènique, 24 % en poids d'unités butadiène-1,2, 28 % en poids d'unités butadiènique-1,4 cis et 48 % en poids d'unités butadiène-1,4 trans par rapport au poids de la partie butadiènique. Sa Mn est égale à 120000 g/mol et son Ip est égal 1,84, ils sont mesurés selon la méthode décrite au paragraphe 3.

On procède de la manière suivante :
On incorpore de l'oxyde de 1-(5-((2-méthyl-1H-imidazol-1-yl)méthyl)furan-2-yl)-N-phénylméthanimine (0,27g, 0,97 mmol) (composé E selon l'invention) ou de l'oxyde de N-(4-((2-méthyl-1H-imidazol-l-yl)méthyl)benzylidène)aniline (Composé E1 non conforme) (0,28 g, 0.97 mmol) à 15 g de SBR tel que décrit ci-dessus sur un outil à cylindres à 23°C. Le mélange est homogénéisé en 15 passes portefeuille. Cette phase de mélangeage est suivie d'un traitement thermique à 160°C pendant 15 minutes sous presse à 10 bars de pression.

On effectue la même expérience pour des temps de greffage de 30 min et de 60 min.

A la fin de chaque expérience, on analyse le taux molaire de greffage du de composé E, conforme à l'invention, ou du composé E1, non conforme à l'invention, est déterminé par RMN ¹H conformément à la méthode décrite au paragraphe 2. Les résultats sont reportés dans le tableau 8.

**[Table 8]**

| | Rendement de greffage (en %) | | |
|---|---|---|---|
| Temps de greffage | 15 min | 30 min | 60 min |
| Composé E | 60 | 67 | 78 |
| Composé E1 | 44 | 46 | 44 |

De manière surprenante, on constate d'après le tableau 8, que le rendement de greffage du composé E conforme à l'invention est significativement toujours amélioré par rapport au composé E1 non conforme. En outre, le rendement de greffage du composé E conforme à l'invention continue à augmenter au-delà des 30 min de réaction alors qu'un plateau est atteint dès 15 min pour le composé E1 non conforme à l'invention.

## Revendications

1. Polymère modifié obtenu par greffage d'au moins un composé de formule (I) sur au moins une insaturation de la chaîne d'un polymère initial 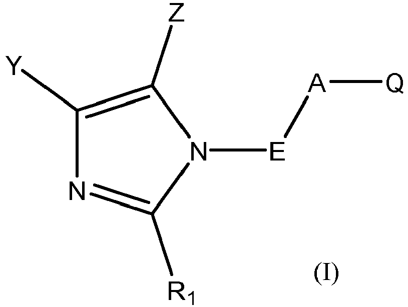 dans laquelle :
- Q représente un dipôle comprenant au moins un atome d'azote ;
- A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ; et
- Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent.

2. Polymère modifié selon la revendication 1, dans lequel le taux molaire de greffage du composé de formule (I) est compris dans un domaine allant de 0,01 à 15%, de préférence de 0,05 à 10%, plus préférentiellement de 0,07 à 5%.

3. Polymère modifié selon la revendication 1 ou 2, dans lequel Q est un groupe de formule (II), (III) ou (IV) 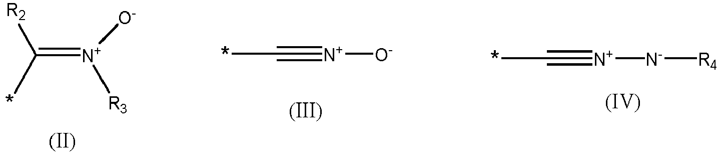 dans lesquelles :
- le symbole * représente le rattachement de Q à A ;
- R₂ et R₄ sont choisis, indépendamment l'un de l'autre, parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

4. Polymère modifié selon la revendication 3, dans lequel le composé de formule (I) est choisi parmi les composés de formule (IIa) 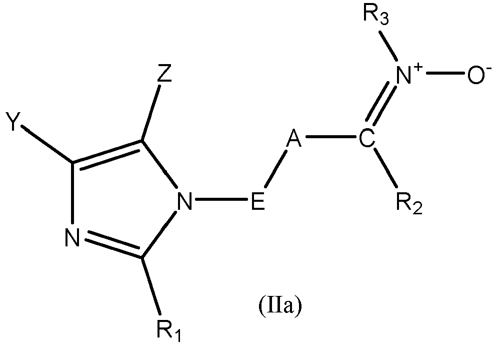 dans laquelle :
- A représente un cycle hétéroaromatique divalent éventuellement substitué par une ou plusieurs chaînes hydrocarbonées, identiques ou différentes, aliphatiques, linéaires ou ramifiées, éventuellement substituées ou interrompues par un ou plusieurs hétéroatomes ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ;
- Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
- R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

5. Polymère modifié selon l'une quelconque des revendications 1 à 4, dans lequel A est un cycle hétéroaromatique divalent formé de 5 à 10 atomes, de préférence de 5 à 6 atomes, éventuellement substitué par une ou plusieurs chaînes hydrocarbonées en C₁-C₂₄, identiques ou différentes, aliphatiques, linéaires ou ramifiées, éventuellement substituées ou interrompues par un ou plusieurs hétéroatomes.

6. Polymère modifié selon la revendication 4 ou 5, dans lequel le composé de formule (IIa) est choisi parmi ceux de la formule (IIb) 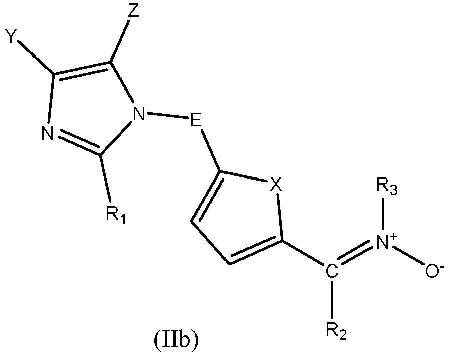 dans laquelle
- X représente un hétéroatome choisi parmi l'atome de soufre, l'atome d'oxygène ou l'atome d'azote, de préférence X est un atome d'oxygène ;
- E représente un groupe divalent hydrocarboné pouvant éventuellement contenir un ou plusieurs hétéroatomes ;
- R₁ représente un atome d'hydrogène ou un groupe alkyle en C₁-C₂₀ ;
- Y et Z, identiques ou différentes, représentent chacun un atome d'hydrogène ou une chaîne hydrocarbonée, Y et Z pouvant également former ensemble un cycle, notamment aromatique, avec les atomes de carbone du cycle imidazole auxquels ils se rattachent ;
- R₂ est choisi parmi un atome d'hydrogène, un alkyle en C₁-C₂₀ linéaire ou ramifié, un cycloalkyle en C₃-C₂₀ éventuellement substitué par une ou plusieurs chaînes hydrocarbonées aliphatiques, de préférence saturées, linéaires ou ramifiées et un aryle en C₆-C₂₀ éventuellement substitué par une ou plusieurs chaînes hydrocarbonées aliphatiques, de préférence saturées, linéaires ou ramifiées ; et
- R₃ est choisi dans le groupe constitué par les alkyles en C₁-C₂₀ linéaires ou ramifiés, les cycloalkyles en C₃-C₂₀ éventuellement substitués par une ou plusieurs chaînes hydrocarbonées aliphatiques, de préférence saturées, linéaires ou ramifiées et les aryles en C₆-C₂₀ éventuellement substitués par une ou plusieurs chaînes hydrocarbonées aliphatiques, de préférence saturées, linéaires ou ramifiées.

7. Polymère modifié selon l'une quelconque des revendications 3 à 6, dans lequel R₂ est un atome d'hydrogène et R₃ est choisi parmi les alkyles en C₁-C₂₀ et les aryles en C₆-C₂₀.

8. Polymère modifié selon l'une quelconque des revendications précédentes, dans lequel E est choisi parmi les chaînes hydrocarbonées aliphatiques, linéaires ou ramifiées, saturées en C₁-C₂₄, préférentiellement en C₁-C₁₀, plus préférentiellement en C₁-C₆ éventuellement interrompues par un ou plusieurs atomes d'azote, de soufre ou d'oxygène.

9. Polymère modifié selon l'une quelconque des revendications précédentes, dans lequel E est choisi parmi les groupes -R-, -NHR-, -OR- et -SR- où R est un alkylène, linéaire ou ramifié, en C₁-C₂₄, de préférence en C₁-C₁₀, plus préférentiellement en C₁-C₆.

10. Polymère modifié selon l'une quelconque des revendications précédentes, dans lequel Y et Z, identiques ou différentes, représentent chacun un atome d'hydrogène ou un alkyle, linéaire ou ramifié, en C₁-C₂₄, de préférence en C₁-C₁₀, plus préférentiellement en C₁-C₆, Y et Z pouvant également former ensemble un cycle, notamment aromatique, avec les atomes de carbone du cycle imidazole auxquels ils se rattachent.

11. Polymère modifié selon l'une quelconque des revendications précédentes, dans lequel Y et Z sont un atome d'hydrogène.

12. Polymère modifié selon l'une quelconque des revendications précédentes, dans lequel R₁ est un atome d'hydrogène ou un alkyle en C₁-C₆, de préférence le méthyle.

13. Polymère modifié selon l'une quelconque des revendications précédentes, dans lequel le composé de formule (I) est le composé de formule (VIII)

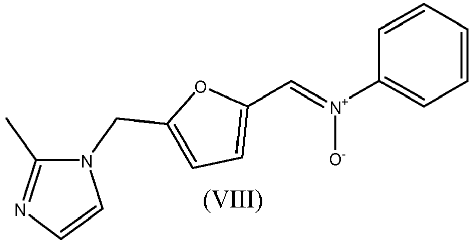

14. Polymère modifié selon l'une quelconque des revendications précédentes, dans lequel le polymère initial est un élastomère, de préférence un élastomère diénique.

15. Composition comprenant au moins un polymère modifié selon l'une quelconque des revendications précédentes 1 à 14 et au moins un additif.

## Patentansprüche

1. Modifiziertes Polymer, erhalten durch Pfropfen mindestens einer Verbindung der Formel (I) auf mindestens eine Ungesättigtheit der Kette eines Ausgangspolymers, 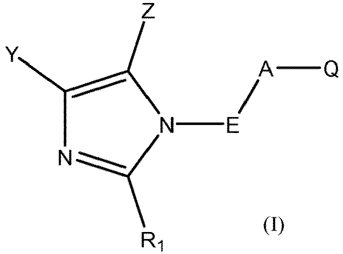 wobei:
- Q für einen Dipol, der mindestens ein Stickstoffatom umfasst, steht;
- A für einen zweiwertigen heteroaromatischen Ring steht, der gegebenenfalls durch eine oder mehrere gleiche oder verschiedene lineare oder verzweigte aliphatische Kohlenwasserstoffketten, die gegebenenfalls durch ein oder mehrere Heteroatome substituiert oder unterbrochen sind, substituiert ist;
- E für eine zweiwertige Kohlenwasserstoffgruppe steht, die gegebenenfalls ein oder mehrere Heteroatome enthalten kann;
- R₁ für ein Wasserstoffatom oder eine C₁-C₂₀-Alkylgruppe steht und
- Y und Z gleich oder verschieden sind und jeweils für ein Wasserstoffatom oder eine Kohlenwasserstoffkette stehen, wobei Y und Z auch zusammen mit den Kohlenstoffatomen des Imidazolrings, an den sie gebunden sind, einen Ring, insbesondere einen aromatischen Ring, bilden können.

2. Modifiziertes Polymer nach Anspruch 1, wobei der molare Pfropfgrad der Verbindung der Formel (I) in einem Bereich von 0,01 bis 15 %, vorzugsweise von 0,05 bis 10 %, weiter bevorzugt von 0,07 bis 5 %, liegt.

3. Modifiziertes Polymer nach Anspruch 1 oder 2, wobei es sich bei Q um eine Gruppe der Formel (II), (III) oder (IV) handelt: 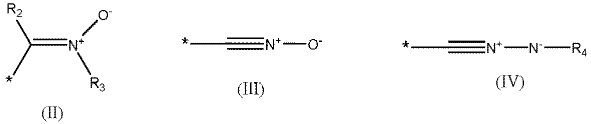 wobei:
- das Symbol * für die Anbindung von Q an A steht;
- R₂ und R₄ unabhängig voneinander aus einem Wasserstoffatom, einem linearen oder verzweigten C₁-C₂₀-Alkyl, einem C₃-C₂₀-Cycloalkyl, das gegebenenfalls durch eine oder mehrere aliphatische Kohlenwasserstoffketten, die vorzugsweise gesättigt, linear oder verzweigt sind, substituiert ist, und einem C₆-C₂₀-Aryl, das gegebenenfalls durch eine oder mehrere aliphatische Kohlenwasserstoffketten, die vorzugsweise gesättigt, linear oder verzweigt sind, substituiert ist, ausgewählt sind und
- R₃ aus der Gruppe bestehend aus linearen oder verzweigten C₁-C₂₀-Alkylgruppen, C₃-C₂₀-Cycloalkylgruppen, die gegebenenfalls durch eine oder mehrere aliphatische Kohlenwasserstoffketten, die vorzugsweise gesättigt, linear oder verzweigt sind, substituiert sind, und C₆-C₂₀-Arylgruppen, die gegebenenfalls durch eine oder mehrere aliphatische Kohlenwasserstoffketten, die vorzugsweise gesättigt, linear oder verzweigt sind, substituiert sind, ausgewählt ist.

4. Modifiziertes Polymer nach Anspruch 3, wobei die Verbindung der Formel (I) aus den Verbindungen der Formel (IIa) ausgewählt ist: 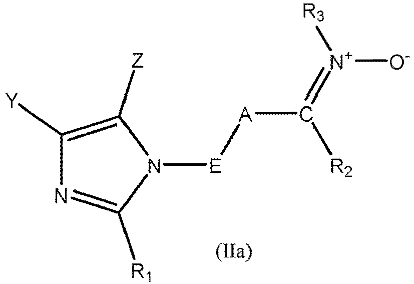 wobei:
- A für einen zweiwertigen heteroaromatischen Ring steht, der gegebenenfalls durch eine oder mehrere gleiche oder verschiedene lineare oder verzweigte aliphatische Kohlenwasserstoffketten, die gegebenenfalls durch ein oder mehrere Heteroatome substituiert oder unterbrochen sind, substituiert ist;
- E für eine zweiwertige Kohlenwasserstoffgruppe steht, die gegebenenfalls ein oder mehrere Heteroatome enthalten kann;
- R₁ für ein Wasserstoffatom oder eine C₁-C₂₀-Alkylgruppe steht und
- Y und Z gleich oder verschieden sind und jeweils für ein Wasserstoffatom oder eine Kohlenwasserstoffkette stehen, wobei Y und Z auch zusammen mit den Kohlenstoffatomen des Imidazolrings, an den sie gebunden sind, einen Ring, insbesondere einen aromatischen Ring, bilden können;
- R₂ aus einem Wasserstoffatom, einem linearen oder verzweigten C₁-C₂₀-Alkyl, einem C₃-C₂₀-Cycloalkyl, das gegebenenfalls durch eine oder mehrere aliphatische Kohlenwasserstoffketten, die vorzugsweise gesättigt, linear oder verzweigt sind, substituiert ist, und einem C₆-C₂₀-Aryl, das gegebenenfalls durch eine oder mehrere aliphatische Kohlenwasserstoffketten, die vorzugsweise gesättigt, linear oder verzweigt sind, substituiert ist, ausgewählt ist und
- R₃ aus der Gruppe bestehend aus linearen oder verzweigten C₁-C₂₀-Alkylgruppen, C₃-C₂₀-Cycloalkylgruppen, die gegebenenfalls durch eine oder mehrere aliphatische Kohlenwasserstoffketten, die vorzugsweise gesättigt, linear oder verzweigt sind, substituiert sind, und C₆-C₂₀-Arylgruppen, die gegebenenfalls durch eine oder mehrere aliphatische Kohlenwasserstoffketten, die vorzugsweise gesättigt, linear oder verzweigt sind, substituiert sind, ausgewählt ist.

5. Modifiziertes Polymer nach einem der Ansprüche 1 bis 4, wobei es sich bei A um einen aus 5 bis 10 Atomen, vorzugsweise 5 bis 6 Atomen, gebildeten zweiwertigen heteroaromatischen Ring handelt, der gegebenenfalls durch eine oder mehrere gleiche oder verschiedene lineare oder verzweigte aliphatische C₁-C₂₄-Kohlenwasserstoffketten, die gegebenenfalls durch ein oder mehrere Heteroatome substituiert oder unterbrochen sind, substituiert ist.

6. Modifiziertes Polymer nach Anspruch 4 oder 5, wobei die Verbindung der Formel (IIa) aus denjenigen der Formel (IIb) ausgewählt ist: 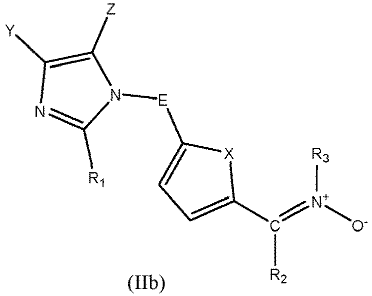 wobei
- X für ein aus einem Schwefelatom, einem Sauerstoffatom oder einem Stickstoffatom ausgewähltes Heteroatom steht, X vorzugsweise ein Sauerstoffatom ist;
- E für eine zweiwertige Kohlenwasserstoffgruppe steht, die gegebenenfalls ein oder mehrere Heteroatome enthalten kann;
- R₁ für ein Wasserstoffatom oder eine C₁-C₂₀-Alkylgruppe steht;
- Y und Z gleich oder verschieden sind und jeweils für ein Wasserstoffatom oder eine Kohlenwasserstoffkette stehen, wobei Y und Z auch zusammen mit den Kohlenstoffatomen des Imidazolrings, an den sie gebunden sind, einen Ring, insbesondere einen aromatischen Ring, bilden können;
- R₂ aus einem Wasserstoffatom, einem linearen oder verzweigten C₁-C₂₀-Alkyl, einem C₃-C₂₀-Cycloalkyl, das gegebenenfalls durch eine oder mehrere aliphatische Kohlenwasserstoffketten, die vorzugsweise gesättigt, linear oder verzweigt sind, substituiert ist, und einem C₆-C₂₀-Aryl, das gegebenenfalls durch eine oder mehrere aliphatische Kohlenwasserstoffketten, die vorzugsweise gesättigt, linear oder verzweigt sind, substituiert ist, ausgewählt ist und
- R₃ aus der Gruppe bestehend aus linearen oder verzweigten C₁-C₂₀-Alkylgruppen, C₃-C₂₀-Cycloalkylgruppen, die gegebenenfalls durch eine oder mehrere aliphatische Kohlenwasserstoffketten, die vorzugsweise gesättigt, linear oder verzweigt sind, substituiert sind, und C₆-C₂₀-Arylgruppen, die gegebenenfalls durch eine oder mehrere aliphatische Kohlenwasserstoffketten, die vorzugsweise gesättigt, linear oder verzweigt sind, substituiert sind, ausgewählt ist.

7. Modifiziertes Polymer nach einem der Ansprüche 3 bis 6, wobei R₂ ein Wasserstoffatom ist und R₃ aus C₁-C₂₀-Alkylgruppen und C₆-C₂₀-Arylgruppen ausgewählt ist.

8. Modifiziertes Polymer nach einem der vorhergehenden Ansprüche, wobei E aus linearen oder verzweigten, gesättigten aliphatischen C₁-C₂₄-, bevorzugt C₁-C₁₀- und weiter bevorzugt C₁-C₆-Kohlenwasserstoffgruppen, die gegebenenfalls durch ein oder mehrere Stickstoff-, Schwefel- oder Sauerstoffatome unterbrochen sind, ausgewählt ist.

9. Modifiziertes Polymer nach einem der vorhergehenden Ansprüche, wobei E aus den Gruppen -R-, -NHR-, -OR- und -SR- ausgewählt ist, wobei R ein lineares oder verzweigtes C₁-C₂₄-, bevorzugt C₁-C₁₀- und weiter bevorzugt C₁-C₆-Alkylen ist.

10. Modifiziertes Polymer nach einem der vorhergehenden Ansprüche, wobei Y und Z gleich oder verschieden sind und jeweils für ein Wasserstoffatom oder ein lineares oder verzweigtes C₁-C₂₄-, bevorzugt C₁-C₁₀- und weiter bevorzugt C₁-C₆-Alkyl stehen, wobei Y und Z auch zusammen mit den Kohlenstoffatomen des Imidazolrings, an den sie gebunden sind, einen Ring, insbesondere einen aromatischen Ring, bilden können.

11. Modifiziertes Polymer nach einem der vorhergehenden Ansprüche, wobei Y und Z ein Wasserstoffatom sind.

12. Modifiziertes Polymer nach einem der vorhergehenden Ansprüche, wobei R₁ ein Wasserstoffatom oder ein C₁-C₆-Alkyl, vorzugsweise Methyl, ist.

13. Modifiziertes Polymer nach einem der vorhergehenden Ansprüche, wobei es sich bei der Verbindung der Formel (I) um die Verbindung der Formel (VIII) handelt:

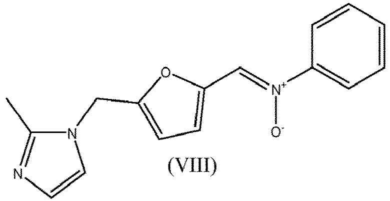

14. Modifiziertes Polymer nach einem der vorhergehenden Ansprüche, wobei es sich bei dem Ausgangspolymer um ein Elastomer, vorzugsweise ein Dienelastomer, handelt.

15. Zusammensetzung, umfassend mindestens ein modifiziertes Polymer nach einem der vorhergehenden Ansprüche 1 bis 14 und mindestens ein Additiv.

## Claims

1. Modified polymer obtained by grafting of at least one compound of formula (I) onto at least one unsaturation of the initial polymer chain 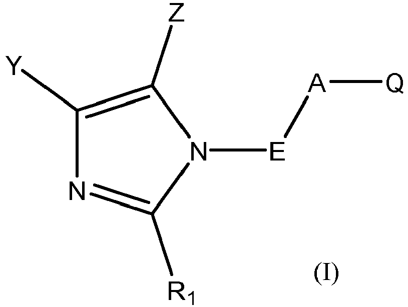 in which:
- Q represents a dipole comprising at least one nitrogen atom;
- A represents a divalent heteroaromatic ring optionally substituted with one or more identical or different linear or branched aliphatic hydrocarbon-based chains, optionally substituted or interrupted with one or more heteroatoms;
- E represents a divalent hydrocarbon-based group which may optionally contain one or more heteroatoms;
- R₁ represents a hydrogen atom or a C₁-C₂₀ alkyl group; and
- Y and Z, which may be identical or different, each represent a hydrogen atom or a hydrocarbon-based chain, Y and Z together also possibly forming a ring, notably an aromatic ring, with the carbon atoms of the imidazole ring to which they are attached.

2. Modified polymer according to Claim 1, in which the molar degree of grafting of the compound of formula (I) is in a range extending from 0.01% to 15%, preferably from 0.05% to 10%, more preferentially from 0.07% to 5%.

3. Modified polymer according to Claim 1 or 2, in which Q is a group of formula (II), (III) or (IV) 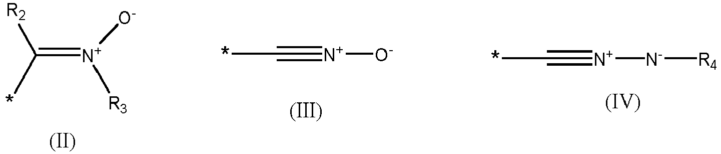 in which:
- the symbol * represents the attachment of Q to A;
- R₂ and R₄ are chosen, independently of each other, from a hydrogen atom, a linear or branched C₁-C₂₀ alkyl, a C₃-C₂₀ cycloalkyl optionally substituted with one or more aliphatic hydrocarbon-based chains, which are preferably saturated, linear or branched, and a C₆-C₂₀ aryl optionally substituted with one or more aliphatic hydrocarbon-based chains, which are preferably saturated, linear or branched; and
- R₃ is chosen from the group consisting of linear or branched C₁-C₂₀ alkyls, C₃-C₂₀ cycloalkyls optionally substituted with one or more aliphatic hydrocarbon-based chains, which are preferably saturated, linear or branched, and C₆-C₂₀ aryls optionally substituted with one or more aliphatic hydrocarbon-based chains, which are preferably saturated, linear or branched.

4. Modified polymer according to Claim 3, in which the compound of formula (I) is chosen from the compounds of formula (IIa) 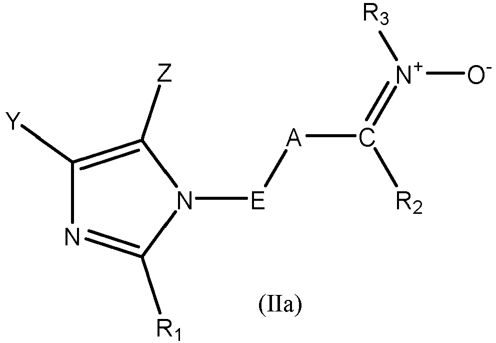 in which:
- A represents a divalent heteroaromatic ring optionally substituted with one or more identical or different linear or branched aliphatic hydrocarbon-based chains, optionally substituted or interrupted with one or more heteroatoms;
- E represents a divalent hydrocarbon-based group which may optionally contain one or more heteroatoms;
- R₁ represents a hydrogen atom or a C₁-C₂₀ alkyl group;
- Y and Z, which may be identical or different, each represent a hydrogen atom or a hydrocarbon-based chain, Y and Z together also possibly forming a ring, notably an aromatic ring, with the carbon atoms of the imidazole ring to which they are attached;
- R₂ is chosen from a hydrogen atom, a linear or branched C₁-C₂₀ alkyl, a C₃-C₂₀ cycloalkyl optionally substituted with one or more aliphatic hydrocarbon-based chains, which are preferably saturated, linear or branched, and a C₆-C₂₀ aryl optionally substituted with one or more aliphatic hydrocarbon-based chains, which are preferably saturated, linear or branched; and
- R₃ is chosen from the group consisting of linear or branched C₁-C₂₀ alkyls, C₃-C₂₀ cycloalkyls optionally substituted with one or more aliphatic hydrocarbon-based chains, which are preferably saturated, linear or branched, and C₆-C₂₀ aryls optionally substituted with one or more aliphatic hydrocarbon-based chains, which are preferably saturated, linear or branched.

5. Modified polymer according to any one of Claims 1 to 4, in which A is a divalent heteroaromatic ring formed from 5 to 10 atoms, preferably from 5 to 6 atoms, optionally substituted with one or more identical or different linear or branched, aliphatic C₁-C₂₄ hydrocarbon-based chains, optionally substituted or interrupted with one or more heteroatoms.

6. Modified polymer according to Claim 4 or 5, in which the compound of formula (IIa) is chosen from those of formula (IIb) 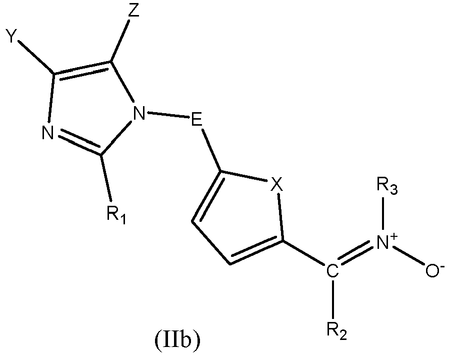 in which:
- X represents a heteroatom chosen from a sulfur atom, an oxygen atom and a nitrogen atom; preferably, X is an oxygen atom;
- E represents a divalent hydrocarbon-based group which may optionally contain one or more heteroatoms;
- R₁ represents a hydrogen atom or a C₁-C₂₀ alkyl group;
- Y and Z, which may be identical or different, each represent a hydrogen atom or a hydrocarbon-based chain, Y and Z together also possibly forming a ring, notably an aromatic ring, with the carbon atoms of the imidazole ring to which they are attached;
- R₂ is chosen from a hydrogen atom, a linear or branched C₁-C₂₀ alkyl, a C₃-C₂₀ cycloalkyl optionally substituted with one or more aliphatic hydrocarbon-based chains, which are preferably saturated, linear or branched, and a C₆-C₂₀ aryl optionally substituted with one or more aliphatic hydrocarbon-based chains, which are preferably saturated, linear or branched; and
- R₃ is chosen from the group consisting of linear or branched C₁-C₂₀ alkyls, C₃-C₂₀ cycloalkyls optionally substituted with one or more aliphatic hydrocarbon-based chains, which are preferably saturated, linear or branched, and C₆-C₂₀ aryls optionally substituted with one or more aliphatic hydrocarbon-based chains, which are preferably saturated, linear or branched.

7. Modified polymer according to any one of Claims 3 to 6, in which R₂ is a hydrogen atom and R₃ is chosen from C₁-C₂₀ alkyls and C₆-C₂₀ aryls.

8. Modified polymer according to any one of the preceding claims, in which E is chosen from linear or branched, saturated aliphatic C₁-C₂₄, preferentially C₁-C₁₀ and more preferentially C₁-C₆ hydrocarbon-based chains optionally interrupted with one or more nitrogen, sulfur or oxygen atoms.

9. Modified polymer according to any one of the preceding claims, in which E is chosen from the groups -R-, -NHR-, -OR- and -SR-, where R is a linear or branched C₁-C₂₄, preferably C₁-C₁₀ and more preferentially C₁-C₆ alkylene.

10. Modified polymer according to any one of the preceding claims, in which Y and Z, which may be identical or different, each represent a hydrogen atom or a linear or branched C₁-C₂₄, preferably C₁-C₁₀ and more preferentially C₁-C₆ alkyl, Y and Z together also possibly forming a ring, notably an aromatic ring, with the carbon atoms of the imidazole ring to which they are attached.

11. Modified polymer according to any one of the preceding claims, in which Y and Z are a hydrogen atom.

12. Modified polymer according to any one of the preceding claims, in which R₁ is a hydrogen atom or a C₁-C₆ alkyl, preferably methyl.

13. Modified polymer according to any one of the preceding claims, in which the compound of formula (I) is the compound of formula (VIII)

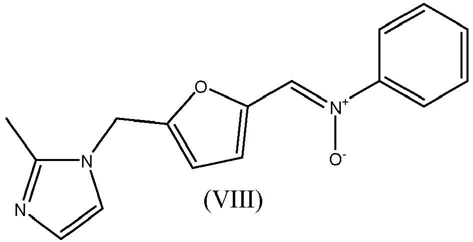

14. Modified polymer according to any one of the preceding claims, in which the initial polymer is an elastomer, preferably a diene elastomer.

15. Composition comprising at least one modified polymer according to any one of the preceding Claims 1 to 14 and at least one additive.
